# EUROPEAN PATENT SPECIFICATION

(11) **EP 0 629 701 B1**
(45) Date of publication and mention of the grant of the patent: **19.01.2000**
(21) Application number: 94108957.5
(22) Date of filing: 10.06.1994
(51) Int. Cl.: C12P 17/02, C12N 9/18, C07D 305/14, C12N 1/20, C12Q 1/04

(54) **Enzymatic hydrolysis methods for the preparation of C-10 and C-13 hydroxyl-bearing taxanes, enzymatic esterification method for the preparation of C-10 acyloxy-bearing taxanes, and use thereof in the preparation of C-13 acyloxy-bearing taxanes**
Enzymatische Hydrolyse oder Esterifikation von C-10 und C-13 Hydroxy oder Acyloxy enthaltende Taxanen
Hydrolyse ou estérification enzymatique de taxanes contenant des substituents C-10 et C-13 hydroxy ou acyloxy

(30) Priority: 15.06.1993 US 77979; 15.06.1993 US 77980
(43) Date of publication of application: 21.12.1994
(73) Proprietor: BRISTOL-MYERS SQUIBB COMPANY, Princeton, NJ 08543-4000 (US)
(72) Inventor: Hanson, Ronald L., Morris Plains, NJ 07950 (US); Patel, Ramesh N., Bridgewater, NJ 08807 (US); Szarka, Laszlo J., East Brunswick, NJ 08816 (US)
(74) Representative: Josif, Albert, Dr.-Ing.

(56) References cited:
- EP-A- 0 253 739
- EP-A- 0 336 840
- EP-A- 0 336 841
- BIOLOGICAL ABSTRACTS, xol. 97 1994, Philadelphia, PA, US; abstract no. 206030, HANSON, R. ET AL. 'Biotransformation of Taxus extracts with site-specific enzymes for hydrolysis of taxanes at C-10 and C-13' & 207 TH NATIONAL MEETING OF THE AMERICAN CHEMICAL SOCIETY,SAN DIEGO,MARCH 13-17,1994.ABSTRACTS OF PAPERS AMERICAN CHEMICAL SOCIETY 207 (1-2) 1994 'CONFERENCE'
- BIOLOGICAL ABSTRACTS, xol. 97 1994, Philadelphia, PA, US; abstract no. 205977, CAZZULINO, D. ET AL. 'Fermentation and recovery of an enzyme used for hydrolysis of taxanes.' & 207 TH NATIONAL MEETING OF THE AMERICAN CHEMICAL SOCIETY, SAN DIEGO, MARCH 13-17,1994 .ABSTRACTS OF PAPERS AMERICAN CHEMICAL SOCIETY 207 (1-2) 1994 'CONFERENCE'

## Description

The present invention relates to an enzymatic hydrolysis method for the preparation of C-10 hydroxyl-bearing taxanes, and an enzymatic esterification method for the preparation of C-10 acyloxy-bearing taxanes, which compounds may be used, for example, as intermediates in the preparation of pharmacologically active taxanes such as taxol and taxol analogues.

The present invention also relates to an enzymatic hydrolysis method for the preparation of C-13 hydroxyl-bearing taxanes useful as intermediates in the preparation of C-13 acyloxy-bearing taxanes, also particularly useful in the preparation of taxol and taxol analogues.

Taxanes are diterpene compounds which find utility in the pharmaceutical field. For example, taxol, a taxane having the structure: 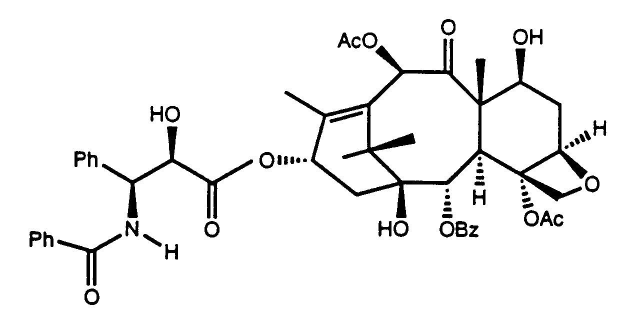 where Ph is phenyl, Ac is acetyl and Bz is benzoyl, has been found to be an effective anticancer agent.

Naturally occurring taxanes such as taxol may be found in plant materials, and have been isolated therefrom. Such taxanes may, however, be present in plant materials in relatively small amounts so that, in the case of taxol, for example, large numbers of the slow-growing yew trees forming a source for the compound may be required. The art has thus continued to search for synthetic, including semi-synthetic routes for the preparation of naturally occurring taxanes such as taxol, as well as for the preparation of analogues thereof.

Due to the complexity of the taxane ring structure, a taxane containing desired substituents on the ring system may more readily be prepared by the use of a starting material already having the basic taxane ring structure. Thus, for example, a compound having the taxane ring structure and containing a hydroxyl group at C-13, and particularly also containing a desired substituent at C-10, may be coupled with an intermediate compound to form a taxane having a desired sidechain at C-13, such as a pharmacologically active taxane having an acyloxy sidechain at C-13 exemplified by taxol or analogues thereof.

The present invention provides methods for obtaining taxanes with desired substituents at C-10. In particular, the present invention provides methods for the preparation of C-10 hydroxyl-bearing, and C-10 acyloxy-bearing taxane compounds, which compounds find utility as starting materials in the preparation of taxanes such as taxol and analogues thereof.

In one embodiment, the present invention provides a method for the preparation of at least one taxane containing a hydroxyl group directly bonded at C-10, comprising the steps of contacting at least one taxane containing an acyloxy group directly bonded at C-10 with an enzyme or microorganism capable of catalyzing the hydrolysis of said acyloxy group to a hydroxyl group, and effecting said hydrolysis.

In another embodiment, the present invention provides a method for the preparation of at least one taxane containing an acyloxy group directly bonded at C-10, comprising the steps of contacting at least one taxane containing a hydroxyl group directly bonded at C-10 with an acylating agent and an enzyme or microorganism capable of catalyzing the esterification of said hydroxyl group to an acyloxy group, and effecting said esterification.

The present invention further provides a method for the preparation of C-13 hydroxyl-bearing taxane compounds, which compounds find utility as starting materials in the preparation of taxanes having a desired sidechain at C-13.

In particular, the present invention provides a method for the preparation of at least one taxane containing a hydroxyl group directly bonded at C-13, comprising the steps of contacting at least one taxane containing an acyloxy group directly bonded at C-13 with an enzyme or microorganism capable of catalyzing the hydrolysis of said acyloxy group to a hydroxyl group, and effecting said hydrolysis.

The present invention provides efficient methods for the preparation of C-10 hydroxyl-bearing taxanes from C-10 acyloxy-bearing taxanes, and for the preparation of C-10 acyloxy-bearing taxanes from C-10 hydroxyl-bearing taxanes. A single taxane may be hydrolyzed, or a mixture of different taxanes may be sequentially or simultaneously hydrolyzed, according to the present invention; likewise, a single taxane may be esterified, or a mixture of different taxanes may be sequentially or simultaneously esterified, according to the present invention.

The present invention further provides an efficient method for the preparation of C-13 hydroxyl-bearing taxanes from C-13 acyloxy-bearing taxanes. A single taxane may be hydrolyzed, or a mixture of different taxanes may be sequentially or simultaneously hydrolyzed, according to the present invention.

The present invention is described further as follows.

### Hydrolysis at C-10

In a preferred embodiment, the present invention provides a method for the preparation of at least one C-10 hydroxyl-bearing taxane of the following formula I: 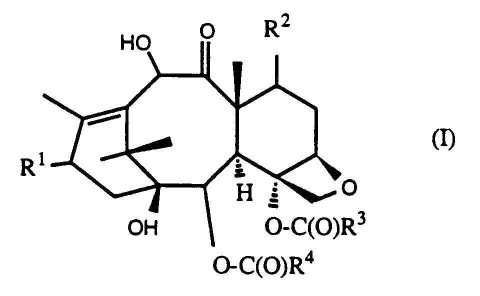 where
- R¹ is: hydroxyl or acyloxy, especially where R¹ has the structure of formula III described below;
- R² is: hydrogen, hydroxyl, fluoro, R⁵-O-, xylosyl, R⁶-C(O)-O- or R⁶-O-C(O)-O-;
- R³ and R⁴: are independently hydrogen, alkyl, alkenyl, alkynyl, cycloalkyl, cycloalkenyl, aryl, or heterocyclo;
- R⁵ is: a hydroxyl protecting group; and
- R⁶ is: hydrogen, alkyl, alkenyl, alkynyl, cycloalkyl, cycloalkenyl, aryl or heterocyclo,
or salts thereof,
comprising the steps of contacting at least one C-10 acyloxy-bearing taxane of the following formula II: 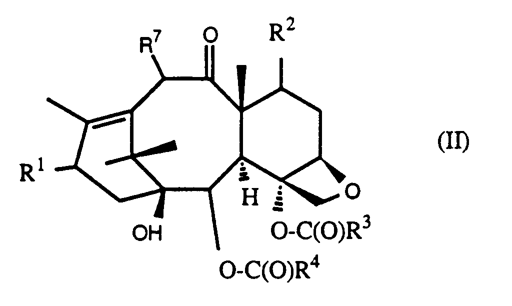 where
R¹, R², R³ and R⁴ are as defined above; and
R⁷ is acyloxy,
   or salts thereof,
   with an enzyme or microorganism capable of catalyzing the hydrolysis of said R⁷ acyloxy group to a hydroxyl group, and effecting said hydrolysis.

All stereoconfigurations of the unspecified chiral centers of the compounds of the formulae I and II are contemplated in the hydrolysis method of the present invention, either alone (that is, substantially free of other stereoisomers) or in admixture with other stereoisomeric forms.

In another preferred embodiment, the present invention provides a method for the preparation of at least one first taxane, having a desired C-10 acyloxy group, from at least one second taxane, having an undesired acyloxy C-10 group, by enzymatic hydrolysis of the latter to provide at least one C-10 hydroxyl-containing analogue by the method described herein, followed by coupling of the desired acyl group thereto to provide the former. In this embodiment, the present invention provides, for example, a method for the preparation of a desired taxane, having a particular C-10 acyloxy group, from a starting mixture of taxanes containing different acyloxy C-10 groups, which starting mixture may or may not include the desired taxane, by simultaneous or sequential hydrolysis of the different C-10 groups to provide one or more taxanes having a hydroxyl group at C-10, followed by coupling of the desired acyl group thereto. This preferred method is particularly useful where a mixture of taxanes having different C-10 acyloxy groups is obtained, such as by extraction of plant materials yielding taxol in admixture with other naturally-produced taxanes, and where a particular taxane such as taxol is ultimately desired. Coupling of the acyl group may be done by non-enzymatic methods for the formation of acyl groups. For example, C-7 protected 10-desacetylbaccatin III (e.g., protected at C-7 by triethylsilyl formed by contacting 10-desacetylbaccatin III with triethylsilylchloride and imidazole in dimethylformamide) may be acylated at C-10 by contact with lithium hexamethyldisilazide in tetrahydrofuran with lithium chloride/acetyl chloride at low temperatures, e.g. -60 to 70°C. Alternatively, coupling of the acyl group may be done by the enzymatic esterification method described herein.

In the method of the present invention, the stereoconfiguration of the C-10 acyloxy group of the starting taxane is preferably retained in the C-10 hydroxyl group-containing product.

### Esterification at C-10

In another preferred embodiment, the present invention provides a method for the preparation of at least one C-10 acyloxy-bearing taxane of the following formula II: 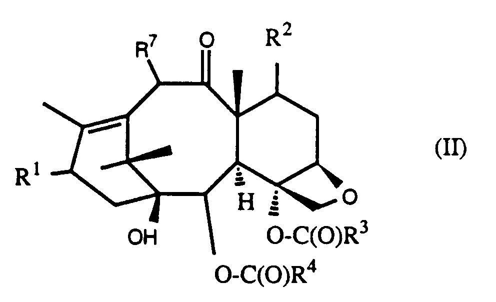 where
- R¹ is: hydroxyl or acyloxy, especially where R¹ has the structure of formula III described below;
- R² is: hydrogen, hydroxyl, fluoro, R⁵-O-, xylosyl, R⁶-C(O)-O- or R⁶-O-C(O)-O-;
- R³ and R⁴: are independently hydrogen, alkyl, alkenyl, alkynyl, cycloalkyl, cycloalkenyl, aryl, or heterocyclo;
- R⁵ is: a hydroxyl protecting group;
- R⁶ is: hydrogen, alkyl, alkenyl, alkynyl, cycloalkyl, cycloalkenyl, aryl or heterocyclo; and
- R⁷ is: acyloxy,
or salts thereof,
comprising the steps of contacting at least one C-10 hydroxyl-bearing taxane of the following formula I: 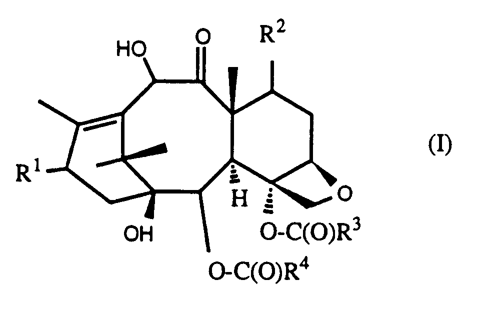 where
R¹, R², R³ and R⁴ are as defined above,
or salts thereof,
   with an acylating agent and an enzyme or microorganism capable of catalyzing the esterification of the C-10 hydroxyl group to form said R⁷ acyloxy group, and effecting said esterification.

All stereoconfigurations of the unspecified chiral centers of the compounds of the formulae I and II are contemplated in the esterification method of the present invention, either alone (that is, substantially free of other stereoisomers) or in admixture with other stereoisomeric forms.

Any acylating agent effecting the esterification of the present invention may be employed. Preferred acylating agents are those of the following formula IV:

R¹¹-C(O)-L (IV)

where
- R¹¹ is: alkyl, alkenyl, alkynyl, aryl, cycloalkyl, cycloalkenyl or heterocyclo; and
- L is: a leaving group which may be displaced to form an ester group.

Preferred R¹¹ groups for the formula IV are alkyl groups such as C₁₋₆ alkyl groups, especially methyl. Exemplary L groups include halogen atoms, hydroxyl, alkoxy, or alkenyloxy groups. Preferred L groups are alkenyloxy groups, most preferably C₁₋₆ alkenyloxy groups such as CH₂=CH-O- and CH₂=C(CH₃)-O-. Isopropenyl acetate and vinyl acetate are particularly preferred acylating agents.

In the method of the present invention, the stereoconfiguration of the C-10 hydroxyl group of the starting taxane is preferably retained in the C-10 acyloxy group-containing product.

### Hydrolysis at C-13

In a preferred embodiment, the present invention provides a method for the preparation of at least one C-13 hydroxyl-bearing taxane of the following formula V: 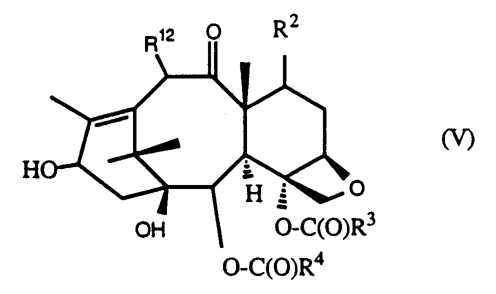 where
- R¹² is: hydrogen, hydroxyl, R⁵-O-, R⁶-C(O)-O-, or R⁶-O-C(O)-O-;
- R² is: hydrogen, hydroxyl, fluoro, R⁵-O-, xylosyl, R⁶-C(O)-O- or R⁶-O-C(O)-O-;
- R³ and R⁴: are independently hydrogen, alkyl, alkenyl, alkynyl, cycloalkyl, cycloalkenyl, aryl, or heterocyclo;
- R⁵ is: a hydroxyl protecting group; and
- R⁶ is: hydrogen, alkyl, alkenyl, alkynyl, cycloalkyl, cycloalkenyl, aryl or heterocyclo,
or salts thereof,
comprising the steps of contacting at least one C-13 acyloxy-bearing taxane of the following formula VI: 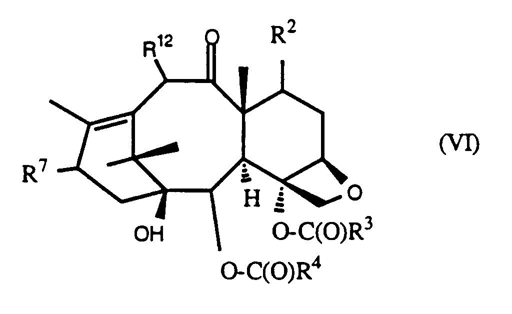 where
R¹², R², R³ and R⁴ are as defined above; and
R⁷ is acyloxy,
   or salts thereof,
   with an enzyme or microorganism capable of catalyzing the hydrolysis of said R⁷ acyloxy group to a hydroxyl group, and effecting said hydrolysis.

All stereoconfigurations of the unspecified chiral centers of the compounds of the formulae V and VI are contemplated in the method of the present invention, either alone (that is, substantially free of other stereoisomers) or in admixture with other stereoisomeric forms.

In another preferred embodiment, the present invention provides a method for the preparation of at least one first taxane, having a desired C-13 acyloxy sidechain, from at least one second taxane, having an undesired acyloxy C-13 sidechain, by enzymatic hydrolysis of the latter to provide at least one C-13 hydroxyl-containing analogue by the method described herein, followed by coupling of the desired sidechain thereto to provide the former. In this embodiment, the present invention provides, for example, a method for the preparation of a desired taxane, having a particular C-13 acyloxy sidechain, from a starting mixture of taxanes containing different acyloxy C-13 sidechains, which starting mixture may or may not include the desired taxane, by simultaneous or sequential hydrolysis of the different C-13 groups to provide one or more taxanes having a hydroxyl group at C-13, followed by coupling of the desired sidechain thereto. This preferred method is particularly useful where a mixture of taxanes having different C-13 acyloxy sidechains is obtained, such as by extraction of plant materials yielding taxol in admixture with cephalomannine and other naturally-produced taxanes, and where a particular taxane such as taxol is ultimately desired.

In the method of the present invention, the stereoconfiguration of the C-13 acyloxy group of the starting taxane is preferably retained in the C-13 hydroxyl group-containing product.

### Definitions

The terms "enzymatic process" or "enzymatic method", as used herein, denote a process or method of the present invention employing an enzyme or microorganism. The term "hydrolysis", as used herein, denotes the formation of a hydroxyl group from an acyloxy group, and may be achieved, for example, by contact with water and/or a suitable organic alcohol according to the method of the present invention. The term "esterification", as used herein, denotes the formation of an acyloxy group from a hydroxyl group. The term "acylating agent", as used herein, denotes a compound capable of effecting the aforementioned esterification by providing an acyl group. Use of "an enzyme or microorganism" in the present methods includes use of two or more, as well as a single, enzyme or microorganism.

The terms "alkyl" or "alk", as used herein alone or as part of another group, denote optionally substituted, straight and branched chain saturated hydrocarbon groups, preferably having 1 to 12 carbons in the normal chain. Exemplary unsubstituted such groups include methyl, ethyl, propyl, isopropyl, n-butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl and the like. Exemplary substituents may include one or more of the following groups: halo, alkoxy, alkylthio, alkenyl, alkynyl, aryl, cycloalkyl, cycloalkenyl, hydroxy or protected hydroxy, carboxyl (-COOH), alkyloxycarbonyl, alkylcarbonyloxy, carbamoyl (NH₂-CO-), amino (-NH₂), mono- or dialkylamino, or thiol (-SH).

The terms "lower alk" or "lower alkyl", as used herein alone or as part of another group, denote such optionally substituted groups as described above for alkyl having 1 to 4 carbon atoms in the normal chain.

The terms "alkoxy" or "alkylthio", as used herein alone or as part of another group, denote an alkyl group as described above bonded through an oxygen linkage (-O-) or a sulfur linkage (-S-), respectively. The term "alkyloxycarbonyl", as used herein alone or as part of another group, denotes an alkoxy group bonded through a carbonyl group. The term "alkylcarbonyloxy", as used herein alone or as part of another group, denotes an alkyl group bonded through a carbonyl group which is, in turn, bonded through an oxygen linkage. The terms "monoalkylamino" or "dialkylamino", as used herein alone or as part of another group, denote an amino group substituted by one or two alkyl groups as described above, respectively.

The term "alkenyl", as used herein alone or as part of another group, denotes such optionally substituted groups as described above for alkyl, further containing at least one carbon to carbon double bond. Exemplary substituents include one or more alkyl groups as described above, and/or one or more groups described above as alkyl substituents. The term "alkenyloxy", as used herein alone or as part of another group, denotes an alkenyl group as described above bonded through an oxygen linkage (-O-).

The term "alkynyl", as used herein alone or as part of another group, denotes such optionally substituted groups as described above for alkyl, further containing at least one carbon to carbon triple bond. Exemplary substituents include one or more alkyl groups as described above, and/or one or more groups described above as alkyl substituents. The term "alkynyloxy", as used herein alone or as part of another group, denotes an alkynyl group as described above bonded through an oxygen linkage (-O-).

The term "cycloalkyl", as used herein alone or as part of another group, denotes optionally substituted, saturated carbocyclic ring systems, preferably containing 1 to 3 rings and 3 to 7 carbons per ring. Exemplary unsubstituted such groups include cyclopropyl, cyclobutyl, cyclopentyl, cyclohexyl, cycloheptyl, cyclooctyl, cyclodecyl, cyclododecyl, and adamantyl. Exemplary substituents include one or more alkyl groups as described above, and/or one or more groups described above as alkyl substituents. The term "cycloalkyloxy", as used herein alone or as part of another group, denotes a cycloalkyl group as described above bonded through an oxygen linkage (-O-).

The term "cycloalkenyl", as used herein alone or as part of another group, denotes such optionally substituted groups as described above for cycloalkyl, further containing at least one carbon to carbon double bond forming a partially unsaturated ring. Exemplary substituents include one or more alkyl groups as described above, and/or one or more groups described above as alkyl substituents. The term "cycloalkenyloxy", as used herein alone or as part of another group, denotes a cycloalkenyl group as described above bonded through an oxygen linkage (-O-).

The terms "ar" or "aryl", as used herein alone or as part of another group, denote optionally substituted, carbocyclic aromatic groups, preferably containing 1 or 2 rings and 6 to 12 ring carbons. Exemplary unsubstituted such groups include phenyl, biphenyl, and naphthyl. Exemplary substituents include one or more, preferably three or fewer, nitro groups, alkyl groups as described above and/or groups described above as alkyl substituents. The term "aryloxy", as used herein alone or as part of another group, denotes an aryl group as described above bonded through an oxygen linkage (-O-).

The terms "heterocyclo" or "heterocyclic", as used herein alone or as part of another group, denote optionally substituted fully saturated or unsaturated, aromatic or non-aromatic cyclic groups having at least one heteroatom in at least one ring, preferably monocyclic or bicyclic groups having 5 or 6 atoms in each ring. The heterocyclo group may, for example, have 1 or 2 oxygen atoms, 1 or 2 sulfur atoms, and/or 1 to 4 nitrogen atoms in the ring. Each heterocyclo group may be bonded through any carbon or heteroatom of the ring system. Exemplary heterocyclo groups include the following: thienyl, furyl, pyrrolyl, pyridyl, imidazolyl, pyrrolidinyl, piperidinyl, azepinyl, indolyl, isoindolyl, quinolinyl, isoquinolinyl, benzothiazolyl, benzoxazolyl, benzimidazolyl, benzoxadiazolyl, and benzofurazanyl. Exemplary substituents include one or more alkyl groups as described above and/or one or more groups described above as alkyl substituents. The term "heterocyclooxy", as used herein alone or as part of another group, denotes a heterocyclo group as described above bonded through an oxygen linkage (-O-).

The terms "halogen" or "halo", as used herein alone or as part of another group, denote chlorine, bromine, fluorine, and iodine.

The term "taxane", as used herein, denotes compounds containing a taxane moiety as described following. The term "taxane moiety", as used herein, denotes moieties containing the core structure (with numbering of ring system positions used herein shown): 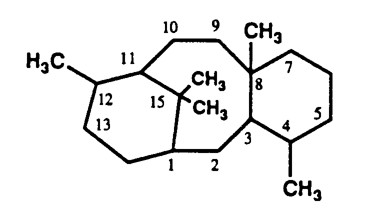 which core structure may be substituted and which may contain ethylenic unsaturation in the ring system thereof. Such moieties having an oxetane ring fused at the 4- and 5-positions, such as is found in taxol, are preferred.

The term "hydroxy (or hydroxyl) protecting group", as used herein, denotes any group capable of protecting a free hydroxyl group which, subsequent to the reaction for which it is employed, may be removed without disturbing the remainder of the molecule. Such groups, and the synthesis thereof, may be found in "Protective Groups in Organic Synthesis" by T.W. Greene, John Wiley and Sons, 1981, or Fieser & Fieser.

The term "salt", as used herein, includes acidic and/or basic salts formed with inorganic and/or organic acids and bases.

The term "acyl", as used herein alone or as part of another group, denotes the moiety formed by removal of the hydroxyl group from the group -COOH of an organic carboxylic acid. The term "acyloxy", as used herein alone or as part of another group, denotes an acyl group as described above bonded through an oxygen linkage (-O-).

### Starting Materials for C-10 Modification

The C-10 acyloxy-bearing, and C-10 hydroxy-bearing taxanes, employed as starting materials for the present invention may be any such compounds capable of undergoing the enzymatic hydrolysis or esterification methods, respectively, of the present invention. The starting materials may be synthetically formed taxanes, or preferably, naturally formed taxanes such as cephalomannine, 7-xylosyltaxol, taxol, baccatin III, 10-desacetylbaccatin III, or taxol C (an analogue of taxol wherein the benzoyl group of the C-13 taxol sidechain is replaced by an n-pentanoyl group), alone or in admixture with each other. The "naturally formed" taxane starting materials are preferably obtained by plant cell culture of, and/or extraction from, taxane-producing plant tissues, particularly tissues from, or derived from, plants of the *Taxus* genus such as *Taxus baccata, Taxus cuspidata, Taxus brevifolia, Taxus wallichiana, Taxus media, Taxus hicksii,* especially *Taxus x*. *media hicksii*. Exemplary plant tissues include the needles, bark and whole seedling.

For preferred methods of obtaining the C-10 hydroxy- and acyloxy-bearing taxane starting materials of the present methods see Rao, *Pharmaceutical Research*, *10*, 521-524 (1993); Kingston, *Pharmac*. *Ther*., 52, 1-34 (1991); or the Examples herein.

### Starting Materials for C-13 Modification

The C-13 acyloxy-bearing taxanes employed as starting materials for the present invention may be any such compounds capable of undergoing the enzymatic hydrolysis of the present invention. The starting materials may be synthetically formed taxanes, or preferably, naturally formed taxanes such as cephalomannine, 7-xylosyltaxol, taxol, 7-xylosyl-10-desacetyltaxol, 10-desacetyltaxol, or taxol C, alone or in admixture with each other. The "naturally formed" taxane starting materials are preferably obtained by plant cell culture of, and/or extraction from, taxane-producing plant tissues, particularly tissues from, or derived from, plants of the *Taxus* genus such as *Taxus baccata, Taxus cuspidata, Taxus brevifolia, Taxus wallichiana, Taxus media, Taxus hicksii,* especially *Taxus x*. *media hicksii*. Exemplary plant tissues include the needles, bark and whole seedling.

For preferred methods of obtaining the C-13 acyloxy-bearing taxane starting materials of the present method see Rao, *Pharmaceutical Research, 10,* 521-524 (1993); Kingston, *Pharmac*. *Ther*., *52,* 1-34 (1991); or the Examples herein.

### Enzymes and Microorganisms for C-10 Modification

The enzyme or microorganism employed in the present invention may be any enzyme or microorganism capable of catalyzing the enzymatic hydrolysis or esterification methods described herein. The enyzmatic or microbial materials, regardless of origin or purity, may be employed in the free state or immobilized on a support such as by physical adsorption or entrapment.

Exemplary microorganisms include those within the following genera: *Nocardioides, Nocardia, Rhodococcus, Micropolyspora, Saccharopolyspora, Pseudonocardia, Oerskovia, Promicromonospora,* and *Intrasporangium*. Particularly preferred microorganisms are those species within the genus *Nocardioides,* such as *Nocardioides albus, Nocardioides flavus, Nocardioides fulvus, Nocardioides luteus, Nocardioides simplex,* and *Nocardioides thermolilacinus,* especially *Nocardioides albus* ATCC 55424 (SC 13910) and ATCC 55425 (SC 13911) and *Nocardioides luteus* ATCC 55426 (SC 13912). The term "ATCC" as used herein refers to the accession number of the American Type Culture Collection, 12301 Parklawn Drive, Rockville, Maryland 20852, the depository for the organism referred to. The above microorganisms ATCC 55424, 55425 and 55426 were deposited on May 12, 1993. The term "SC" denotes the designation given to the microorganism as part of the Squibb culture collection.

The biologically pure microorganisms *Nocardioides albus* ATCC 55424 (SC 13910), *Nocardioides albus* ATCC 55425 (SC 13911), and *Nocardioides luteus* ATCC 55426 (SC 13912) are novel microorganisms. It should be understood that mutants of these organims are also contemplated by the present invention, for use in the hydrolysis or esterification methods described herein, such as those modified by the use of chemical, physical (for example, X-rays) or biological means (for example, by molecular biology techniques).

*Nocardioides albus* ATCC 55424 (SC 13910) and ATCC 55425 (SC 13911) may be cultivated on Medium A 94 (corn steep liquor (35 grams), Cerelose (20 grams), (NH₄)₂SO₄ Reagent Grade (5 grams), CaCO₃ (3.5 grams), soy bean oil (5 ml) and distilled water (1 liter)). These organisms were isolated from soil (from a sample from New Brunswick, New Jersey), and are gram positive, non-motile organisms exhibiting aerobic growth on a variety of media. On solid YS medium (0.2% yeast extract, 1% starch), the mycelium is whitish to light cream colored. Growth is associated with production of a dark diffusible pigment in both solid and liquid media. Microscopically, growth in liquid culture is characterized by mycelial aggregates consisting of abundantly branching hyphae.

*Nocardioides luteus* ATCC 55426 (SC 13912) may be cultivated on Medium A 94 (corn steep liquor (35 grams), Cerelose (20 grams), (NH₄)₂SO₄ Reagent Grade (5 grams), CaCO₃ (3.5 grams), soy bean oil (5 ml) and distilled water (1 liter)). This organism was isolated from soil (from a sample from New Brunswick, New Jersey), and is a gram positive, non-motile organism exhibiting aerobic growth on a variety of media. On solid YS medium (0.2% yeast extract, 1% starch), the mycelium is dark cream colored. Microscopically, growth in liquid culture is characterized by mycelial aggregates consisting of abundantly branching hyphae.

The above organisms *Nocardioides albus* ATCC 55424 (SC 13910) and ATCC 55425 (SC 13911), and *Nocardioides luteus* ATCC 55426 (SC 13912), were identified as strains of *Nocardioides albus* and *Nocardioides luteus*, respectively, in accordance with the description given in *Bergey's Manual* of *Systematic Bacteriology*, Volume 2 (Ed. P.H.A. Sneath) (1986).

Exemplary enzymes for use in the present hydrolysis or esterification methods are hydrolases, particularly esterases, proteases or lipases. Preferred enzymes include those derived from microorganisms, particularly those microorganisms described above. Enzymes may be isolated, for example, by extraction and purification methods, such as by use of hydrophobic interaction chromatography, gel filtration, followed by an anion exchange column. The present invention further provides the enzymes capable of the present hydrolysis or esterification methods which may be isolated from *Nocardioides albus* ATCC 55424 (SC 13910) and ATCC 55425 (SC 13911), and *Nocardioides luteus* ATCC 55426 (SC 13912), for example, by the above techniques.

### Enzymes and Microorganisms for C-13 Modification

The enzyme or microorganism employed in the present invention may be any enzyme or microorganism capable of catalyzing the enzymatic hydrolysis described herein. The enyzmatic or microbial materials, regardless of origin or purity, may be employed in the free state or immobilized on a support such as by physical adsorption or entrapment.

A preferred method for selecting a microorganism suitable for enzymatic hydrolysis of a starting C-13 acyloxy-bearing taxane according to the method of the present invention is by use of the following novel screening method, comprising the steps of:
(a) selecting a solid growth medium (i) in which the microorganism to be screened will grow, (ii) in which the starting C-13 acyloxy-bearing taxane is insoluble and thus, in admixture with the growth medium, has a cloudy appearance, and (iii) in which the C-13 hydroxyl-bearing taxane product of the hydrolysis method of the present invention is soluble, and, preferably, in which the cleaved C-13 sidechain is also soluble, and thus, in admixture with the growth medium, has a clear appearance;
(b) placing the microorganism into contact with the growth medium selected in step (a) above, for example, in a petri dish, into which the starting C-13 acyloxy-bearing taxane has been admixed, and under conditions allowing growth of the microorganism to occur; and
(c) observing whether a clear zone appears around the area in which growth of the microorganism occurs.

The formation of a clear zone indicates that hydrolysis has occurred, and thus that the microorganism may be suitable for use in the present hydrolysis method. As used herein, the terms "clear" and "cloudy" are to be construed relative to each other. Thus, the starting C-13 acyloxy-bearing taxane is employed in admixture with the growth medium in sufficient quantity so as to be visible in suspension therein (giving a "cloudy" appearance), and clarity is determined relative to said initial degree of visibility in suspension, that is, as a lessening of that visibility.

Another preferred screening method provided by the present invention is that corresponding to the method set forth above, with the exception that a solid growth medium is selected in which the starting C-13 acyloxy-bearing taxane is soluble and thus, in admixture with the growth medium, has a clear appearance, while the C-13 hydroxyl-bearing taxane product of the hydrolysis method of the present invention is insoluble (and preferably in which the cleaved C-13 sidechain is also insoluble) and thus, in admixture with the growth medium, has a cloudy appearance. Observance of a cloudy zone around the area in which growth of the microorganism occurs allows selection of a suitable microorganism.

A particularly preferred embodiment of the screening method of the present invention is that set forth in the Examples herein.

The above preferred screening methods may suitably be employed where the starting C-13 acyloxy-bearing taxane and the C-13 hydroxyl-bearing taxane product differ in relative solubility to the extent that it may be observed whether or not hydrolysis according to the present invention has occurred.

Exemplary microorganisms for use in C-13 hydrolysis include those within the following genera: *Nocardioides, Nocardia, Rhodococcus, Micropolyspora, Saccharopolyspora, Pseudonocardia, Oerskovia, Promicromonospora,* and *'Intrasporangium*. Particularly preferred microorganisms are those species within the genus *Nocardioides,* such as *Nocardioides albus, Nocardioides flavus, Nocardioides fulvus, Nocardioides luteus, Nocardioides simplex,* and *Nocardioides thermolilacinus,* especially *Nocardioides albus* ATCC 55424 (SC 13910) and ATCC 55425 (SC 13911) and *Nocardioides luteus* ATCC 55426 (SC 13912).

As described above, the biologically pure microorganisms *Nocardioides albus* ATCC 55424 (SC 13910), *Nocardioides albus* ATCC 55425 (SC 13911), and *Nocardioides luteus* ATCC 55426 (SC 13912) are novel microorganisms further provided by the present invention. It should be understood that mutants of these organisms are also contemplated by the present invention, for use in the hydrolysis methods described herein, such as those modified by the use of chemical, physical (for example, X-rays) or biological means (for example, by molecular biology techniques).

Exemplary enzymes for use in the present method are hydrolases. Preferred enzymes include those derived from microorganisms, particularly those microorganisms described above. Enzymes may be isolated, for example, by extraction and purification methods such as those described in the Examples herein, especially purification of the active fractions found extracellularly in the medium in which these organisms have been cultivated, such as by use of an anion exchange column, followed by hydrophobic interaction chromatography and gel filtration. The present invention further provides the enzymes capable of the present hydrolysis which may be isolated from *Nocardioides albus* ATCC 55424 (SC 13910) and ATCC 55425 (SC 13911), and *Nocardioides luteus* ATCC 55426 (SC 13912), for example, by the above techniques.

### Use of Enzymes and Microorganisms in C-10 and C-13 Modifications

Where microorganisms are employed, the cells may be used in the form of intact wet cells or dried cells such as lyophilized, spray-dried or heat-dried cells, or in the form of treated cell material such as ruptured cells or cell extracts. The use of genetically engineered organisms is also contemplated. The host cell may be any cell, e.g. *Escherichia coli,* modified to contain a gene or genes for expressing one or more enzymes capable of catalysis as described herein.

Where one or more microorganisms are employed, the enzymatic hydrolysis or esterification methods of the present invention may be carried out subsequent to the fermentation of the microorganism (two-stage fermentation and hydrolysis or esterification), or concurrently therewith, that is, in the latter case, by *in situ* fermentation and hydrolysis or esterification (single-stage fermentation and hydrolysis or esterification).

Growth of the microorganisms may be achieved by one of ordinary skill in the art by the use of an appropriate medium. Appropriate media for growing microorganisms include those which provide nutrients necessary for the growth of the microbial cells. A typical medium for growth includes necessary carbon sources, nitrogen sources, and elements (e.g. in trace amounts). Inducers may also be added. The term "inducer", as used herein, includes any compound enhancing formation of the desired enzymatic activity within the microbial cell.

Carbon sources may include sugars such as maltose, lactose, glucose, fructose, glycerol, sorbitol, sucrose, starch, mannitol, propylene glycol, and the like; organic acids such as sodium acetate, sodium citrate, and the like; and alcohols such as ethanol, propanol and the like.

Nitrogen sources may include N-Z amine A, corn steep liquor, soy bean meal, beef extracts, yeast extracts, molasses, baker's yeast, tryptone, nutrisoy, peptone, yeastamin, amino acids such as sodium glutamate and the like, sodium nitrate, ammonium sulfate and the like.

Trace elements may include magnesium, manganese, calcium, cobalt, nickel, iron, sodium and potassium salts. Phosphates may also be added in trace, or preferably, greater than trace amounts.

The medium employed may include more than one carbon or nitrogen source or other nutrient.

Preferred media for growth include aqueous media, particularly those described in the Examples herein.

The agitation and aeration of the reaction mixture affects the amount of oxygen available during the hydrolysis or esterification process when conducted, for example, in shake-flask cultures or fermentor tanks during growth of microorganisms. The agitation range from 100 to 250 RPM is preferred; aeration of about 1 to 10 volumes of air per volume of media per minute is preferred.

For growth of the microorganisms and/or hydrolysis or esterification according to the method of the present invention, the pH of the medium is preferably from about 6 to about 8.5, and the temperature is preferably from about 24°C to about 37°C. Hydrolysis or esterification may, for example, be carried out *in vitro* over time periods such as 1 to 48 hours, or preferably until the yield of desired product is maximized. It is preferred to conduct the hydrolysis methods of the present invention at a pH of from 6 to 8, particularly under non-basic conditions.

It is also preferred to employ an aqueous liquid as the hydrolysis reaction medium, although an organic liquid, or a miscible or immiscible (biphasic) organic/aqueous liquid mixture, may also be employed. It is preferred to employ an organic solvent or biphasic organic/aqueous reaction medium for esterification, although other media may be employed.

For C-10 modification, it is preferred to employ 0.025 to 2.5 weight % of the C-10 hydroxy- or acyloxy-bearing taxane starting material(s) based on the combined weight of starting material(s) and esterification or hydrolysis reaction medium. In the esterification method of the present invention, preferred molar ratios of acylating agent to C-10 hydroxyl-bearing taxane are from about 1:1 to about 1000:1. The amount of enzyme or microorganism employed relative to the starting material is selected to allow catalysis of the enzymatic hydrolysis or esterification of the present invention. It is preferred to obtain yields in excess of 90% (% C-10 hydrolyzed product obtained based on the starting acyloxy-bearing taxane) or in excess of 50% (% C-10 acylated product obtained based on the starting hydroxyl-bearing taxane) when employing the hydrolysis or esterification methods of the present invention, respectively. Hydrolysis or esterification may be obtained selectively at C-10 of the starting taxane. That is, product(s) the greater portion (such as solely) of which are hydrolyzed or esterified at C-10 only may be obtained without hydrolysis or esterification at other positions.

For C-13 modification, it is preferred to employ 0.025 to 0.25 weight % of the C-13 acyloxy-bearing taxane starting material(s) based on the combined weight of starting material(s) and hydrolysis reaction medium. The amount of enzyme or microorganism employed relative to the starting material is selected to allow catalysis of the enzymatic hydrolysis of the present invention. It is preferred to obtain yields in excess of 90% (% C-13 hydrolyzed product obtained based on the starting acyloxy taxane) when employing the hydrolysis method of the present invention. Hydrolysis may be obtained selectively at C-13 of the starting taxane. That is, product(s) the greater portion (such as solely) of which are hydrolyzed at C-13 only may be obtained without hydrolysis at other positions.

### Separation

The C-10 acyloxy- or hydroxyl-bearing products of the processes of the present invention, and coupled products such as those described below, may be isolated and purified, for example, by methods such as extraction, distillation, crystallization, and column chromatography.

Similarly, the C-13 hydroxyl-bearing products of the C-13 hydrolysis process of the present invention, and coupled products such as those described below, may also be isolated and purified, for example, by methods such as extraction, distillation, crystallization, and column chromatography.

### Utility

Taxanes are diterpene compounds containing a taxane moiety as described above. Of particular interest are taxanes containing a taxane moiety in which the 11,12-positions are bonded through an ethylenic linkage, and in which the 13-position contains a sidechain, which taxanes are exemplified by taxol. Pharmacologically active taxanes such as taxol may be used as antitumor agents to treat patients suffering from cancers such as breast, ovarian, colon or lung cancers, melanoma and leukemia.

### Compounds Obtained by C-10 Modification

The compounds obtained by the C-10 hydrolysis or esterification methods of the present invention are particularly useful as intermediates in the preparation of the aforementioned pharmacologically active taxanes by allowing preparation of compounds having a desired substituent at C-10. Thus, for example, where the compounds prepared by C-10 modification according to the methods of the present invention also bear a hydroxyl group at C-13, such compounds may be coupled with C-13 acyloxy sidechain-forming intermediate compounds, such as β-lactams, to obtain C-13 acyloxy sidechain-bearing taxanes such as taxol or analogues thereof. In this regard, modification at C-13 according to the methods of the present invention may be conducted prior to, during, or after the methods of the present invention for modification at C-10 are employed.

The acyloxy- or hydroxyl-bearing compounds prepared according to the methods of the present invention may optionally be modified prior to use in C-13 acyloxy sidechain coupling. For example, one or more hydroxyl groups at positions other than C-13 may be protected prior to coupling and, thereafter, deprotected.

The C-10 acyloxy- and hydroxyl-bearing taxanes obtained by the hydrolysis and esterification methods of the present invention, optionally modified as above, may, for example, be used in the preparation of C-13 acyloxy sidechain-bearing taxanes such as those recited, and prepared by the methods described in, European Patent Publication No. 400,971, U.S. Patent No. 4,876,399, U.S. Patent No. 4,857,653, U.S. Patent No. 4,814,470, U.S. Patent No. 4,924,012, U.S. Patent No. 4,924,011, and Kingston, *Pharm. Ther.,* Vol. 52, 1 - 34 (1991), especially U.S. Patent Application Serial No. 07/995,443, filed December 23, 1992 by Poss et al. (Attorney Docket No. LD60) and U.S. Patent Application Serial No. 08/033,598, filed March 19, 1993 by Thottathil et al. (Attorney Docket No. LD57), all incorporated herein by reference.

### Compounds Obtained by C-13 Modification

The C-13 hydroxyl containing compounds obtained by the hydrolysis method of the present invention are particularly useful as intermediates in the preparation of the aforementioned C-13 sidechain-bearing taxanes. In particular, the C-13 hydroxyl-bearing compounds prepared according to the present method may be coupled with sidechain-forming intermediate compounds, such as β-lactams, to obtain the C-13 sidechain-bearing taxanes. The addition of such a sidechain, in and of itself, may impart an increased or more desirable pharmacological activity to the taxane product, or may form a taxane product which is more readily converted to a taxane having an increased or more desirable pharmacological activity than the starting compound.

The C-13 hydroxyl-bearing compounds prepared according to the method of the present invention may optionally be modified prior to use in sidechain formation by coupling. For example, modification at C-10 according to the methods of the present invention may be conducted prior to, during, or subsequent to the C-13 hydrolysis method of the present invention; and/or one or more hydroxyl groups at positions other than C-13 may be protected prior to coupling and, thereafter, deprotected.

The C-13 hydroxyl-bearing taxanes obtained by the hydrolysis method of the present invention, optionally modified as above, may, for example, be used in the preparation of C-13 acyloxy sidechain-bearing taxanes such as those recited, and prepared by the methods described in, European Patent Publication No. 400,971, U.S. Patent No. 4,876,399, U.S. Patent No. 4,857,653, U.S. Patent No. 4,814,470, U.S. Patent No. 4,924,012, U.S. Patent No. 4,924,011, and Kingston, *Pharm. Ther*., Vol. 52, 1 - 34 (1991), especially U.S. Patent Application Serial No. 07/995,443, filed December 23, 1992 by Poss et al. (Attorney Docket No. LD60) and U.S. Patent Application Serial No. 08/033,598, filed March 19, 1993 by Thottathil et al. (Attorney Docket No. LD57), all incorporated herein by reference. Preparation of C-13 acyloxy-bearing taxanes of the formula VI is preferred.

### Preferred Compounds for C-10 Modification

It is preferred to employ taxanes of the formula II or salts thereof in the C-10 hydrolysis method of the present invention, whereby enzymatic hydrolysis provides the corresponding compounds of the formula I or salts thereof. It is likewise preferred to employ taxanes of the formula I or salts thereof in the C-10 esterification method of the present invention, whereby enzymatic esterification provides the corresponding compounds of the formula II or salts thereof.

In formulae I and II, R⁷ is preferably R¹¹-C(O)-O-, especially where R¹¹ is alkyl such as methyl; R² is preferably hydroxyl or xylosyl; R³ is preferably alkyl such as methyl; R⁴ is preferably aryl such as phenyl; and R¹ is preferably hydroxyl or a group of the following formula III: 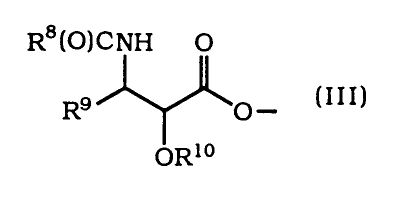 where
- R⁸ and R⁹: are independently alkyl, alkoxy, alkenyl, alkenyloxy, alkynyl, alkynyloxy, cycloalkyl, cycloalkyloxy, cycloalkenyl, cycloalkenyloxy, aryl, aryloxy, heterocyclo or heterocyclooxy; and
- R¹⁰ is: hydrogen or a hydroxyl protecting group.

Exemplary taxanes of the formulae I and II are cephalomannine, 10-desacetyltaxol, 7-xylosyltaxol, taxol-C, 7-xylosyl-10-desacetyltaxol, taxol, baccatin III, 10-desacetylbaccatin III, 7-xylosylbaccatin III, and 7-xylosyl-10-desacetylbaccatin III. Enzymatic hydrolysis of baccatin III to form 10-desacetylbaccatin III (e.g., with the formation of acetic acid), for example, employing hydrolase, is a preferred embodiment of the present invention. This reaction may be reversed via the enzymatic esterification method of the present invention.

Taxol is preferably ultimately prepared by the methods described herein.

### Preferred Compounds for C-13 Modification

It is preferred to employ taxanes of the formula VI or salts thereof in the method of the present invention, whereby enzymatic hydrolysis at C-13 provides the corresponding compounds of the formula V or salts thereof. In formulae V and VI, R¹² is preferably R⁶-C(O)-O- such as acetyloxy, or hydroxyl; R² is preferably hydroxyl or xylosyl; R³ is preferably alkyl such as methyl; R⁴ is preferably aryl such as phenyl; and R⁷ is preferably a group of the following formula III: 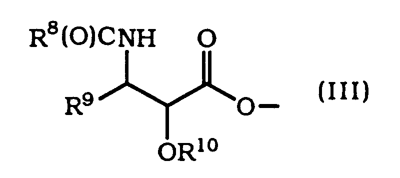 where
R⁸, R⁹ and R¹⁰ are as defined above.

Exemplary starting taxanes of the formula VI are cephalomannine, 10-desacetyltaxol, 7-xylosyltaxol, taxol-C, and 7-xylosyl-10-desacetyltaxol, alone or in admixture with each other or taxol. Preferred hydrolysis products are baccatin III, 10-desacetylbaccatin III, 7-xylosylbaccatin III, and 7-xylosyl-10-desacetylbaccatin III.

Coupling subsequent to hydrolysis preferably provides taxane products of the formula VI described above having C-13 acyloxy groups of the formula III. Taxol is preferably ultimately prepared by hydrolysis and coupling as described herein.

Salts or solvates such as hydrates of reactants or products may be employed or prepared as appropriate in any of the methods of the present invention.

The present invention is further described by the following examples which are illustrative only, and are in no way intended to limit the scope of the instant claims.

### Example 1

### 10-Deacetylation of Baccatin III

*Nocardioides luteus* ATCC 55426 (SC 13912) isolated from soil was grown for three days at 28^{o}C, 150 rpm in a 50 ml Erlenmeyer flask containing 10 ml medium. The medium contained per liter distilled water: 10 g Bacto tryptone, 5 g Bacto yeast extract, 6 ml tributyrin, and 0.06 ml Tween 80 at a final pH of 6.8 ± 0.2. Cells were harvested by centrifugation, washed with 10 ml 50 mM potassium phosphate buffer pH 7, and resuspended in 2 ml of this buffer. 0.5 mg baccatin III in 20 µl methanol was added to the cell suspension and the suspension was mixed for 20 hours at ambient temperature (about 23^{o}C) with a Fisher Roto-Rack. The suspension was extracted with methylene chloride, and the extract was evaporated, redissolved and assayed by HPLC Method 2 described following. The sample contained 0.257 mg/ml 10-desacetylbaccatin III (100% conversion) and only a trace of baccatin III. Washed cells of the strain ATCC 55426 grown on three other media also carried out this transformation.

### Example 2

### 10-Deacetylation of Baccatin III

*Nocardioides luteus* ATCC 55426 (SC 13912) grown in 20 ml medium containing per liter distilled water: 10 g Bacto tryptone, 5 g Bacto yeast extract and 0.06 ml Tween 80 at a pH of 6.8 ± 0.2 was used to inoculate 1 L of the same medium in a 4 L Erlenmeyer flask. The flask was shaken for three days at 28^{o}C, then cells were harvested by centrifugation. The cell pellet was washed with 600 ml 50 mM potassium phosphate buffer pH 7 and centrifuged again to give 36.6 g wet cells. The cells were frozen at -72^{o}C, lyophilized to 2.5 g in 2 days, ground with a mortar and pestle and stored at 2^{o}C. 1.98 ml 50 mM potassium phosphate buffer pH 7, 50 mg dried cells, and 0.5 mg baccatin III in 20 µl methanol were mixed with a Roto-Rack at ambient temperature for the times indicated on Table 1 following. Reactions were stopped by addition of 2 ml methanol. Precipitate was removed with a microfuge, and samples were assayed by HPLC Method 1 described following. The results obtained are shown in Table 1.

**Table 1**

| **Time (Min)** | **10-desacetylbaccatin III mg/ml** | **Baccatin III mg/ml** | **Conversion (%)** |
|---|---|---|---|
| 0 | 0.007 | 0.252 | - |
| 30 | 0.051 | 0.143 | 22 |
| 60 | 0.106 | 0.106 | 46 |
| 120 | 0.204 | 0.034 | 88 |

### Example 3

### 10-Deacetylation of Taxol

A partially purified extract from *Nocardioides luteus* ATCC 55426 (SC 13912) (purified by anion exchange chromatography on Whatman DE52) contained 34 milliunits/ml enzyme. (1 milliunit is the amount of enzyme able to hydrolyze 1 nmole baccatin III to 10-desacetylbaccatin III per minute at 28°C in 50 mM potassium phosphate buffer pH 7 containing 0.25 mg/ml baccatin III and 1% methanol.) 2 ml containing 50 mM potassium phosphate buffer pH 7, 0.5 mg taxol, 1% methanol, and 34 milliunits enzyme was incubated on a Fisher RotoRack for 3.5 hours at 28°C. The reaction was stopped by extraction with 4 ml methylene chloride. The extract was dried, redissolved in methanol and assayed by HPLC Method 3 described following. The sample contained 0.075 mg taxol and 0.186 mg/ml 10-desacetyltaxol (78% conversion).

### Alternative Purification of Nocardioides luteus ATCC 55426 (SC 13912)

*Nocardioides luteus* ATCC 55426 was grown in a fermentor on a medium containing 1% tryptone and 0.5% yeast extract. All purification steps were carried out at 4°C in 50 mM phosphate buffer pH 7.2. The cells were suspended at 10% w/v in buffer and passed through a micro-fluidizer twice at 10,000 psi for lysis. The cell lysate was then clarified by centrifugation at 24,000 x g for 15 min. Ammonium sulfate was added to 60% saturation and the resulting precipitate was suspended in buffer containing 1M ammonium sulfate and applied to a hydrophobic interaction chromatography (HIC-ether (Toya-pearl)) column (5.5 x 2.6 cm) at a flow rate of 2 ml/min. The enzyme activity was eluted with buffer. The active fractions were then loaded on a Pharmacia Sephacryl S-200 gel filtration column (2.6 x 84 cm) at 0.5 ml/min. The active fractions from the column were loaded on an anion exchange (BioRad-Q2) column (2 ml column, flow rate 2 ml/min) and the activity was eluted with a salt gradient of 0-0.8M NaCl in 42 ml. The active fractions were pooled and applied to a Sephacryl S-200 column as described above. The molecular weight of the enzyme was estimated to be 40,000 ± 10,000 on gels containing sodium dodecyl sulfate. The results obtained at various steps of the above method were as follows:

| Step* | Volume ml | Protein mg | Activity mu | Specific Activity mu/mg | Recovery % |
|---|---|---|---|---|---|
| Cell extract | 100 | 450 | 2050 | 4.5 | - |
| Amm. Sulfate | 70 | 196 | 2830 | 14.4 | 138 |
| HIC (ether) | 7 | 24 | 953 | 39.9 | 46.5 |
| Sephacryl S-200 | 7 | 0.7 | 190 | 271 | 9.3 |
| BioRad-Q2 | 3 | 0.09 | 51 | 567 | 2.5 |
| Sephacryl S-200 | 8 | 0.03 | 26 | 867 | 1.3 |

| | | | | | |
|---|---|---|---|---|---|
| * Purification of C-10-deacetylase: 1 milliunit enzyme (mu) catalyzes the conversion of 1 nmole/min of baccatin-III to 10-deacetylbaccatin-III at 25°C in 50 mM potassium phosphate buffer, pH 7, containing 0.25 mg/ml baccatin-III and 1% methanol. Protein was determined with BioRad Protein Assay Reagent. | | | | | |

### Example 4

### Acetylation of 10-Desacetylbaccatin III to Baccatin III

*Nocardioides luteus* ATCC 55426 (SC 13912) cells in 50 mM potassium phosphate buffer pH 7.2 (10% weight/volume) were disrupted with a microfluidizer and the 10-deacetylase enzyme was adsorbed from the extract on Whatman DEAE cellulose DE52 anion exchanger by stirring for 3 hours (10 g extract protein per liter DE52). The DE52 was collected by filtration, washed with buffer and lyophilized to give 0.091 milliunits enzyme per mg solid. 0.2 ml 1 M potassium phosphate buffer pH 8, 100 mg immobilized enzyme (i.e., immobilized on the DE52 resin), 1.8 ml water, 2 mg 10-desacetylbaccatin III and 0.5 ml vinyl acetate were vigorously stirred with a magnetic bar for 14.5 hours at room temperature. The reaction mixture was extracted with methylene chloride. The extract was dried and redissolved in methanol for analysis by HPLC Method 1 described following. The sample contained 0.688 mg/ml 10-desacetylbaccatin III and 0.203 mg/ml baccatin III (19% conversion).

### HPLC Methods

### Method 1

- Column:: Hewlett Packard hypersil 5 micron ODS C18 200 x 4.6 mm
- Mobile phase:: 55% methanol, 45% water
- Flow rate:: 1 ml/min
- Column temperature:: ambient
- Detection wavelength:: 235 nm

### Method 2

Column: Phase Separations Inc. (Norwalk, CT) microbore spherisorb phenyl 150 x 2.0 mm, 3 micron
Mobile phase: Solvent A:15 mM KH₂PO₄, adjusted to pH 4 with trifluoroacetic acid. Solvent B: acetonitrile

| **Time/Minute** | **Solvent A (%)** | **Solvent B (%)** |
|---|---|---|
| 0 | 75 | 25 |
| 20 | 55 | 45 |
| 23 | 40 | 60 |
| 24 | 25 | 75 |
| 28 | 75 | 25 |

Column temperature: 35°C
Detection wavelength: 230 nm.
¹ See also Monsarrat et al., *Drug Metabolism and Disposition*, *18*, 895-901 (1990).

### Method 3¹

- Column:: Hewlett Packard hypersil 5 micron ODS C18 200 x 4.6 mm
- Mobile phase:: 60% methanol, 40% water
- Flow Rate:: 1 ml/min
- Column temperature:: ambient
- Detection wavelength:: 235 nm

### Example 5

### Hydrolysis of Seedling Extract

An ethanol extract of *Taxus hicksii* seedlings was concentrated 10- to 15-fold with nanofilters. 0.5 ml extract, 0.5 ml 1 M potassium phosphate buffer pH 7, 54 milliunits partially purified enzyme from *Nocardioides albus* ATCC 55425 (SC 13911) (purified by anion exchange chromatography and ammonium sulfate precipitation) and 4 ml water were incubated at 28°C for 48 hours on a Fisher Roto Rack. (An enzyme from ATCC 55425 may be employed for the hydrolysis at C-13 as described herein). A second tube contained the same mixture and also 14 milliunits enzyme from *Nocardioides luteus* ATCC 55426 (SC 13912) immobilized on 500 mg DE52 as described in Example 4. After 23 hours incubation at 28°C, a second portion of 14 milliunits enzyme from ATCC 55426 on DE52 was added along with 4 ml water, and incubation was continued for 22 hours. A control sample received no enzymes. Samples were extracted with methylene chloride, and the evaporated extracts were dissolved in methanol for analysis by HPLC Method 2 described above. The results obtained are shown in Table 2 following.

The results show that, after treatment with the C-13 deacylase from SC 13911, there was depletion of taxol, cephalomannine, 7-xylosyl-10-deacetyltaxol and 10-deacetyltaxol, while the amount of baccatin III and 10-deacetylbaccatin III increased. The molar ratios of 10-deacetylbaccatin III plus baccatin III to initial taxol was increased from 117% to 436%. When the seedling extract was treated with both the C-13 deacylase from SC 13911 and the C-10 deacylase from SC 13912, baccatin III was converted to 10-deacetylbaccatin III and the concentration of 10-deacetylbaccatin III was increased by 6-fold compared to the initial value. Thus, treatment of a mixture of taxanes with the C-13 deacylase and C-10 deacylase gave 10-deacetylbaccatin III as the principle product.

**Table 2**

| Hydrolysis of Seedling Extract | | | | | | |
|---|---|---|---|---|---|---|
| Enzyme from | 10-desacetylbaccatin III mg/ml | baccatin III mg/ml | 7-xylosyl-10-desacetyltaxol mg/ml | cephalomannine mg/ml | taxol mg/ml | 10-DAT mg/ml |
| None | 0.167 | 0.142 | 0.246 | 0.243 | 0.401 | 0.201 |
| SC13911 | 0.400 | 0.771 | 0.027 | 0.000 | 0.000 | 0.014 |
| 11 +12⁺ | 1.025 | 0.000 | 0.000 | 0.000 | 0.000 | 0.000 |

| Enzyme from | taxol C mg/ml | D+B/T * | | | | |
|---|---|---|---|---|---|---|
| None | trace | 116.8 | | | | |
| SC13911 | 0.000 | 436.3 | | | | |
| 11 + 12 | 0.000 | 400.8 | | | | |
| 10-DAT is 10-desacetyltaxol | | | | | | |

| | | | | | | |
|---|---|---|---|---|---|---|
| ⁺ SC 13911 and SC 13912 | | | | | | |
| * D+B/T is the molar % yield of (10-deacetylbaccatin III and baccatin III) divided by (initial taxol) | | | | | | |

### Example 6

### Selection of Strains of Microorganisms Capable of Removing the C-13 Sidechain of Taxol

Difco spirit blue agar medium (5.25 g) in 150 ml distilled water was sterilized and partially cooled according to the manufacturer's instructions. The medium contained 10 g tryptone, 5 g yeast extract, 20 g agar, and 0.15 g spirit blue per liter. 25 mg taxol and 10 µl Tween 80 in 1 ml methanol were added to the medium through a sterile filter. 15 ml medium were used per 100 mm x 15 mm petri dish.

After the plates had cooled and dried, soil samples were plated out as follows. 2 g soil was suspended in 40 ml water. A sample of the suspension was diluted 100-fold with water and 0.1 ml was spread per plate. The water used had been filtered through a 0.22 µ filter. Plates were incubated at 28^{o}C, and colonies surrounded by a cleared zone were selected. The basis for the selection was that taxol is insoluble at the concentration used in the medium, giving the plates a cloudy appearance, whereas baccatin III and the side chain produced by hydrolysis are soluble, thereby forming a clear zone around the colonies. The microorganisms selected include: *Nocardioides albus* strains ATCC 55424 (SC 13910) and ATCC 55425 (SC 13911); and *Nocardioides luteus* strain ATCC 55426 (SC 13912).

### Example 7

### Removal of C-13 Sidechain from Taxol

The reaction of this Example proceeded as set forth in Scheme 1 following. 50 ml Erlenmeyer flasks containing 10 ml medium were inoculated with *Nocardioides albus* strains ATCC 55424 (SC 13910) or ATCC 55425 (SC 13911) isolated from soil as described in Example 6 and shaken for two days at 28^{o}C, 150 RPM. The medium contained per liter distilled water: 10 g Bacto tryptone, 5 g Bacto yeast extract and 0.06 ml Tween 80 at pH 6.8 ± 0.2. Cells were removed by centrifugation and the pH of the supernatants was adjusted from 8.66 to 7 with 1 M KH₂PO₄.

0.5 mg taxol in 20 µl methanol was added to 2 ml of each supernatant and the mixtures were stirred for 21 hours at ambient temperature (about 23^{o}C). The solutions were extracted with 4 ml CH₂Cl₂. Extracts were dried under N₂ at room temperature, redissolved in methanol and analyzed by HPLC (Method 3 described above). Supernatant from strain ATCC 55424 (SC 13910) gave 0.008 mg/ml taxol and 0.163 mg/ml baccatin III (94.9 mol% conversion). Supernatant from strain ATCC 55425 (SC 13911) gave 0.014 mg/ml taxol and 0.166 mg/ml baccatin III (96.7 mol% conversion). (Baccatin III and the cleaved side chain product were identified by LC-MS).

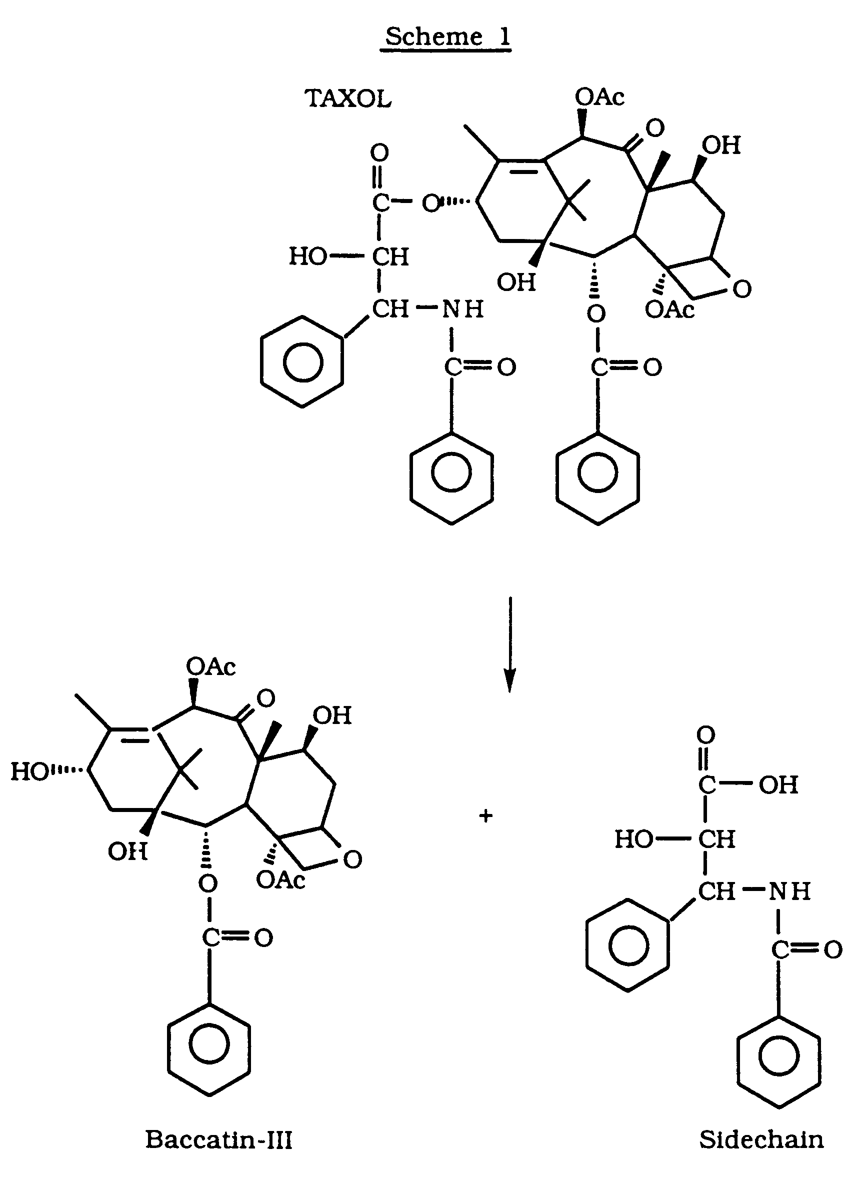

### Example 8

### Removal of C-13 Sidechain from Cephalomannine

Two 50 ml Erlenmeyer flasks each containing 10 ml medium (35 g corn steep liquor, 20 g cerelose, 5 g (NH₄)₂SO₄, 3.5 g CaCO₃, and 5 ml soybean oil brought to 1 liter with distilled water) were each inoculated with 0.5 ml *Nocardiodes albus* strain ATCC 55425 (SC 13911). After two days incubation at 28^{o}C, 150 RPM, these two cultures were added to a 4 L flask containing 1 L of the medium used in Example 7. After 72 hours at 28^{o}C, 200 RPM, cells were removed by centrifugation and the supernatant was adjusted from pH 8.16 to pH 7 with 1 M KH₂PO₄.

0.5 mg cephalomannine in 20 µl methanol was incubated with 2 ml supernatant for 17 hours on an end-over-end shaker (Fisher Roto-Rack). The solution was extracted with methylene chloride, the extract was evaporated, resuspended in methanol and analyzed by HPLC (Method 2 described above). Cephalomannine concentration was decreased from 0.175 mg/ml to 0 and 0.110 mg/ml baccatin III was produced (89 mol% yield based on analyzed initial cephalomannine concentration).

### Example 9

### Removal of C-13 Sidechain from 7-Xylosyltaxol

Enzymes from *Nocardiodes albus* strains ATCC 55424 (SC 13910) and ATCC 55425 (SC 13911) were prepared as described in Example 8, except that 1 L cultures were incubated for two days instead of three. *Nocardioides luteus* strain ATCC 55426 (SC 13912) was grown as described fo*r Nocardiodes albus* strain ATCC 55425 (SC 13911) in Example 8. After three days, cells were harvested by centrifugation, washed with 600 ml 50 mM potassium phosphate buffer pH 7 and centrifuged again. 36.6 g wet cells were frozen at -72^{o}C, lyophilized to 2.52 g in two days, ground with a mortar and pestle, and stored at 2^{o}C.

1.8 ml supernatant from strains ATCC 55424 (SC 13910) and ATCC 55425 (SC 13911), and 50 mg dried cells of strain ATCC 55426 (SC 13912) in 1.8 ml water were each incubated with 0.5 mg 7-xylosyltaxol in 0.2 ml methanol for 42 hours at room temperature on a Fisher Roto-Rack. The reactions were extracted with methylene chloride, and the dried extracts were dissolved in methanol for HPLC analysis (Method 3 described above). 7-Xylosyltaxol decreased from 0.218 mg/ml to 0 with each enzyme. The major product in each sample was identified as 7-xylosylbaccatin III by HPLC - mass spectrometry. Based on a baccatin III standard, ATCC 55424 (SC 13910), ATCC 55425 (SC 13911), and ATCC 55426 (SC 13912) gave 7-xylosylbaccatin III product concentrations of 0.112, 0.118, and 0.120 mg/ml, respectively. Using the same extinction coefficient at 235 nm for baccatin III and 7-xylosylbaccatin III, the calculated yields would be 88%, 93%, and 94%, respectively (molar %s).

### Example 10

### Hydrolysis of Plant Cell Mixtures

1 g *Taxus hicksii* needles was extracted with 10 ml methanol:acetic acid 5000:1. To 10 ml extract was added 10 ml water:acetic acid 500:1. The mixture was centrifuged for 10 minutes at 48,000 x g and 20^{o}C. The pellet was discarded. 4 ml extract and 16 ml supernatant from *Nocardioides* *albus* strain ATCC 55424 (SC 13910) or ATCC 55425 (SC 13911) (prepared as described in Example 9) were shaken in a 50 ml Erlenmeyer flask at 28^{o}C, 150 RPM for 42 hours. Samples were extracted with methylene chloride and the evaporated extracts were dissolved in methanol for analysis by HPLC (Method 2 described above). The taxane concentrations and molar ratios of (baccatin III + 10-desacetylbaccatin III)/initial taxol, with and without enzyme treatment, are shown in the following Table 3.

**Table 3**

| Enzymatic Hydrolysis of Needle Extract | | | | | | |
|---|---|---|---|---|---|---|
| Enzyme from⁺ | 10-desacetylbaccatin III µg/ml | baccatin III µg/ml | 7-xylosyl-10-desacetyltaxol µg/ml | cephalomannine µg/ml | taxol µg/ml | (DAB+B)* (taxol) |
| None | 0.126 | trace | 0.012 | 0.153 | 0.392 | 50.4 |
| SC13910 | 0.602 | 0.319 | 0.000 | 0.000 | 0.000 | 359.3 |
| SC13911 | 0.959 | 0.294 | 0.000 | 0.000 | 0.000 | 492.8 |

| | | | | | | |
|---|---|---|---|---|---|---|
| * Values given in molar % (DAB + B)÷(initial taxol); | | | | | | |
| DAB = 10-desacetylbaccatin III B = baccatin III | | | | | | |

### Example 11

### Purification of Enzyme and Hydrolysis of Taxanes

100 ml medium (35 g corn steep liquor, 20 g cerelose, 5 g ammonium sulfate, 3.5 g calcium carbonate, and 5 ml soybean oil brought to 1 liter with distilled water) in a 500 ml Erlenmeyer flask was inoculated with 1 ml *Nocardioides albus* ATCC 55425 (SC 13911) in the same medium and shaken for 2 days at 28°C. 20 ml of this culture was transferred to 1 L distilled water containing 10 g tryptone and 5 g yeast extract, pH 6.8 ± 0.2 in a 4 L Erlenmeyer flask. The flask was shaken for 3 days at 28°C, cells were removed by centrifugation and the supernatant was adjusted from pH 7.89 to pH 7 with 1M KH₂PO₄.

All purification steps were performed at 4°C. The supernatant was applied to 150 ml Whatman DE52 anion exchanger in 25 mM potassium phosphate buffer pH 7 in a 5 cm diameter column. The column was washed with 150 ml buffer, then 450 ml buffer containing 0.25 M NaCl. Enzyme activity was eluted with buffer plus 0.45 M NaCl. The flow rate was 5 ml/min. Ammonium sulfate (100 mg/ml) was added to the most active fractions from the DE52 column, and the solution was applied to 30 ml Pharmacia Phenyl Sepharose CL-4B (hydrophobic interaction chromatography) in 50 mM potassium phosphate buffer pH 7 containing 100 mg/ml ammonium sulfate in a 2.6 cm diameter column at a flow rate of 1.6 ml/min. The column was washed with 150 ml buffer plus 20 mg/ml ammonium sulfate, then activity was eluted with buffer alone. The most active fractions from the Phenyl Sepharose column were concentrated by ultrafiltration to 4 ml using an Amicon YM10 membrane and passed through a Pharmacia Sephacryl S-200 gel filtration column (2.6 x 84 cm) at a flow rate of 0.65 ml/min. The fraction of highest specific activity had a single band of molecular weight 49,000 ± 10,000 on a sodium dodecyl sulfate (SDS) gel. The purification is summarized in Table 4 following. (1 milliunit enzyme (mu) catalyzes the conversion of 1 nmole/min of taxol to baccatin-III at 28°C in 50 mM potassium phosphate buffer containing 0.25 mg/ml taxol and 1% methanol.)

**Table 4**

| Step | Volume ml | Protein mg | Activity mu |
|---|---|---|---|
| medium | 1000 | 885 | 4664 |
| DE52 | 99 | 104 | 1194 |
| Phenyl Sepharose CL-4B | 16 | 6.45 | 801 |
| Sephacryl S-200 | 8.8 | 0.150 | 164 |

| Step | Specific Activity mu/mg | | Recovery % |
|---|---|---|---|
| medium | 5.27 | | |
| DE52 | 11.5 | | 25.6 |
| Phenyl Sepharose CL-4B | 124 | | 17.2 |
| Sephacryl S-200 | 1093 | | 3.5 |

To demonstrate the activity of the enzyme purified above, the following experiments were conducted employing the taxane substrates listed in Table 5.

Samples were prepared containing 0.5 mg taxane substrate and 1% methanol in 2.0 ml 50 mM potassium phosphate buffer, pH 7 (Samples 1, 2, 3, 4 and 5). Samples were also prepared containing 10 mu enzyme of the enzyme purified through the Phenyl Sepharose CL-4B step described above in addition to 0.5 mg taxane substrate and 1% methanol in 2.0 ml 50 mM potassium phosphate buffer, pH 7 (Samples 1e, 2e, 3e, 4e and 5e). All samples were mixed for 16 hours with a Fisher Roto Rack at 28°C. Samples 1, 1e, 2, 2e, 5 and 5e were extracted with 4 ml methylene chloride. 2 ml of the extract were dried, redissolved in 1 ml methanol and analyzed by HPLC Method 3. Samples 3, 3e, 4 and 4e were diluted with 2 ml methanol and analyzed by HPLC Method 3 (C-13 sidechain was measured in these latter samples only).

Table 5 shows the amount of taxane substrate (mg/ml) remaining in each of the samples after incubation, as well as the amount of product taxane (mg/ml) present at the end of the incubation. As can be seen from Table 5 (see Samples 1e, 2e, 3e, 4e and 5e), the enzyme purified as above is effective in removing the C-13 side chain by hydrolysis of the taxane substrates listed therein.

**Table 5**

| Hydrolysis of Taxanes by Purified Enzyme | | | | | |
|---|---|---|---|---|---|
| Sample | Substrate | Remaining mg/ml | Product | mg/ml | Side chain mg/ml |
| 1 | taxol | 0.268 | | 0.003 | |
| 1e | | 0.030 | baccatin-III | 0.170 | |
| 2 | cephalomannine | 0.239 | | | |
| 2e | | 0.011 | baccatin-III | 0.190 | |
| 3 | 7-xylosyltaxol | 0.250 | | | |
| 3e | | 0.016 | 7-xylosylbaccatin-III | 0.182 | 0.066 |
| 4 | 7-xylosyl-10-deacetyltaxol | 0.250 | | | |
| 4e | | 0.113 | 7-xylosyl-10-deacetylbaccatin-III | 0.108 | 0.044 |
| 5 | 10-deacetyltaxol | 0.257 | | | |
| 5e | | 0.000 | 10-deacetyl baccatin-III | 0.150 | |

### Example 12

### Hydrolysis of Seedling Extract

An ethanol extract of *Taxus hicksii* seedlings was concentrated 10- to 15-fold with nanofilters. 0.5 ml extract, 0.5 ml 1 M potassium phosphate buffer pH 7, 54 mu partially purified enzyme from *Nocardioides albus* ATCC 55425 (SC 13911) (see Example 11 above) and 4 ml water were mixed at 28°C for 48 hours on a Fisher Roto Rack. A control sample received no enzyme. Samples were extracted with methylene chloride and the evaporated extracts were dissolved in methanol for analysis by HPLC Method 2. The results obtained are shown in the following Table 6.

**Table 6**

| Hydrolysis of Seedling Extract | | | | | |
|---|---|---|---|---|---|
| Enzyme from | 10-DAB mg/ml | bacc-III mg/ml | ceph mg/ml | XDT mg/ml | taxol mg/ml |
| none | 0.167 | 0.142 | 0.243 | 0.246 | 0.401 |
| SC13911 | 0.400 | 0.771 | 0.000 | 0.027 | 0.000 |

| Enzyme from | 10-DAT mg/ml | taxol C mg/ml | (DAB+B)/(initial taxol) mol% yield | | |
|---|---|---|---|---|---|
| none | 0.201 | trace | 116.8 | | |
| SC13911 | 0.014 | 0.000 | 436.3 | | |

### In Table 6:

10-DAB is 10-desacetylbaccatin III
bacc-III or B is baccatin III
ceph is cephalomannine
XDT is 7-xylosyl-10-desacetyltaxol
10-DAT is 10-desacetyltaxol

## Claims

1. A method for the preparation of at least one taxane containing a hydroxyl group directly bonded at C-10, comprising the steps of contacting at least one taxane containing an acyloxy group directly bonded at C-10 with an enzyme or microorganism capable of catalyzing the hydrolysis of said acyloxy group to a hydroxyl group, and effecting said hydrolysis.

2. The method of claim 1, wherein at least one C-10 hydroxyl-bearing taxane of the following formula I is prepared: 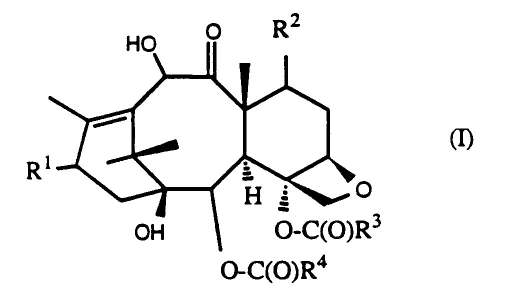 where
R¹ is hydroxyl or acyloxy;
R² is hydrogen, hydroxyl, fluoro, R⁵-O-, xylosyl, R⁶-C(O)-O- or R⁶-O-C(O)-O-;
R³ and R⁴ are independently hydrogen, alkyl, alkenyl, alkynyl, cycloalkyl, cycloalkenyl, aryl, or heterocyclo;
R⁵ is a hydroxyl protecting group; and
R⁶ is hydrogen, alkyl, alkenyl, alkynyl, cycloalkyl, cycloalkenyl, aryl or heterocyclo,
or a salt thereof,
by contacting at least one C-10 acyloxy-bearing taxane of the following formula II: 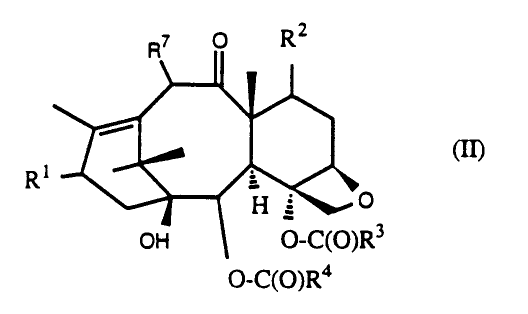 where
R¹, R², R³ and R⁴ are as defined above; and
R⁷ is acyloxy,
or a salt thereof,
with an enzyme or microorganism capable of catalyzing the hydrolysis of said R⁷ acyloxy group to a hydroxyl group.

3. The method of claim 1, wherein the acyloxy-bearing taxane starting material employed in said hydrolysis method comprises a mixture of acyloxy-bearing taxanes.

4. A method for the preparation of at least one taxane containing an acyloxy group directly bonded at C-10, comprising the steps of contacting at least one taxane containing a hydroxyl group directly bonded at C-10 with an acylating agent and an enzyme or microorganism capable of catalyzing the esterification of said hydroxyl group to an acyloxy group, and effecting said esterification.

5. The method of claim 4 , wherein at least one C-10 acyloxy-bearing taxane of the following formula II is prepared: 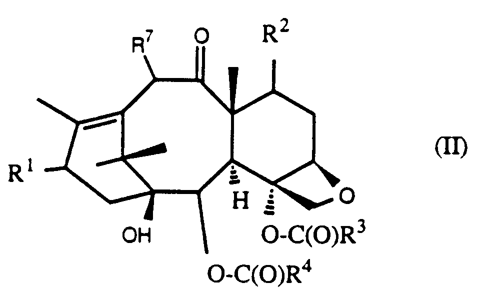 where
R¹ is hydroxyl or acyloxy;
R² is hydrogen, hydroxyl, fluoro, R⁵-O-, xylosyl, R⁶-C(O)-O- or R⁶-O-C(O)-O-;
R³ and R⁴ are independently hydrogen, alkyl, alkenyl, alkynyl, cycloalkyl, cycloalkenyl, aryl, or heterocyclo;
R⁵ is a hydroxyl protecting group;
R⁶ is hydrogen, alkyl, alkenyl, alkynyl, cycloalkyl, cycloalkenyl, aryl or heterocyclo; and
R⁷ is acyloxy,
or a salt thereof,
by contacting at least one C-10 hydroxyl-bearing taxane of the following formula I: 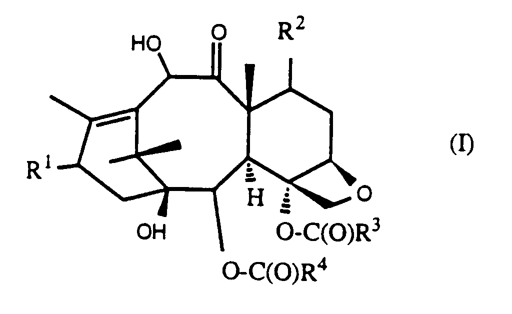 where
R¹, R², R³ and R⁴ are as defined above,
or a salt thereof,
with an acylating agent and an enzyme or microorganism capable of catalyzing the esterification of the C-10 hydroxyl group to form said R⁷ acyloxy group.

6. The method of claim 4 , wherein the hydroxy-bearing taxane starting material employed in said esterification method comprises a mixture of hydroxy-bearing taxanes.

7. The method of claim 6 , wherein said mixture of taxanes is obtained by plant cell culture of, and/or extraction from, plant tissue, wherein said plant is a member of the *Taxus* genus.

8. The method of claim 4, wherein said acylating agent is a compound of the following formula IV:
R¹¹-C(O)-L (IV)
where
R¹¹ is alkyl, alkenyl, alkynyl, aryl, cycloalkyl, cycloalkenyl or heterocyclo; and
L is a leaving group which may be displaced to form an ester group.

9. The method of claim 8, wherein said acylating agent is vinyl acetate.

10. An enzyme capable of catalyzing the hydrolysis of said acyloxy group in the method of claim 1, or capable of catalyzing the esterification of said hydroxyl group to an acyloxy group in the method of claim 4, said enzyme being isolated from *Nocardioides luteus* ATCC 55426 (SC 13912).

11. A method for the preparation of at least one taxane containing a hydroxyl group directly bonded at C-13, comprising the steps of contacting at least one taxane containing an acyloxy group directly bonded at C-13 with an enzyme or microorganism capable of catalyzing the hydrolysis of said acyloxy group to a hydroxyl group, and effecting said hydrolysis.

12. The method of claim 11, wherein at least one C-13 hydroxyl-bearing taxane of the following formula V is prepared: 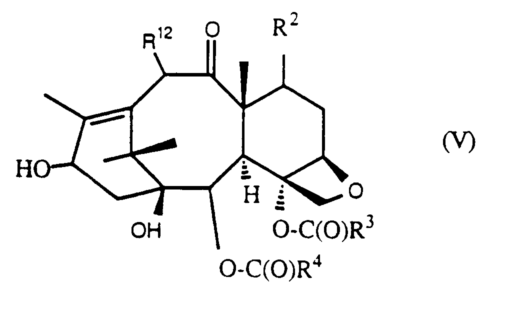 where
R¹² is hydrogen, hydroxyl, R⁵-O-, R⁶-C(O)-O-, or R⁶-O-C(O)-O-;
R² is hydrogen, hydroxyl, fluoro, R⁵-O-, xylosyl, R⁶-C(O)-O- or R⁶-O-C(O)-O-;
R³ and R⁴ are independently hydrogen, alkyl, alkenyl, alkynyl, cycloalkyl, cycloalkenyl, aryl, or heterocyclo;
R⁵ is a hydroxyl protecting group; and
R⁶ is hydrogen, alkyl, alkenyl, alkynyl, cycloalkyl, cycloalkenyl, aryl or heterocyclo,
or a salt thereof,
by contacting at least one C-13 acyloxy-bearing taxane of the following formula VI: 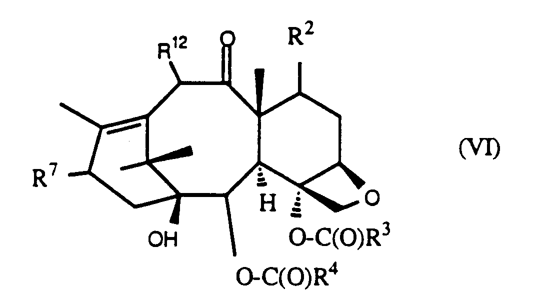 where
R¹², R², R³ and R⁴ are as defined above; and
R⁷ is acyloxy,
or a salt thereof,
with said enzyme or microorganism.

13. The method of claim 12, wherein said taxane of the formula VI is cephalomannine, 7-xylosyl-10-desacetyltaxol, 10-desacetyltaxol, 7-xylosyltaxol, taxol-C, and/or taxol, and said taxane of the formula V is baccatin III, 10-desacetylbaccatin III, 7-xylosyl-10-desacetylbaccatin III and/or 7-xylosylbaccatin III.

14. The method of claim 12, wherein the acyloxy-bearing taxane starting material employed in said hydrolysis method comprises a mixture of acyloxy-bearing taxanes having different sidechains at C-13.

15. The method of claim 11, wherein said enzyme is a hydrolase.

16. The method of claim 11, wherein, subsequent to said hydrolysis, said at least one taxane containing a hydroxyl group directly bonded at C-13, in which hydroxyl groups at positions other than C-13 are optionally protected, is coupled with a compound forming an acyloxy sidechain at C-13.

17. The method of claim 16, wherein taxol is ultimately prepared by said method comprising hydrolysis and coupling.

18. The method of claim 2, wherein said taxane of the formula II is baccatin III, and wherein said taxane of the formula I is 10-desacetylbaccatin III.

19. The method of claims 3 or 14, wherein said mixture of taxanes is obtained by plant cell culture of, and/or extraction from, plant tissue, wherein said plant is a member of the *Taxus* genus.

20. The method of anyone of claims 1,4 and 11, wherein a microorganism is employed which is within one of the following genera: *Nocardioides, Nocardia, Rhodococcus, Micropolyspora, Saccharopolyspora, Pseudonocardia, Oerskovia, Promicromonospora,* or *Intrasporangium*.

21. The method of claim 20, wherein a microorganism is employed which is within the genus *Nocardioides*.

22. The method of claim 21, wherein said microorganism is selected from the group consisting of *Nocardioides albus, Nocardioides flavu*s, *Nocardioides fulvus, Nocardioides luteus, Nocardioides simplex,* and *Nocardioides thermolilacinus*.

23. The method of claim 22, wherein said microorganism is selected from the group consisting of *Nocardioides albus* ATCC 55424 (SC 13910), *Nocardioides albus* ATCC 55425 (SC 13911), and *Nocardioides luteus* ATCC 55426 (SC 13912).

24. The method of anyone of claims 1,4 and 11, wherein said enzyme is derived from a microorganism which is within one of the following genera: *Nocardioides*, *Nocardia, Rhodococcus, Micropolyspora, Saccharopolyspora, Pseudonocardia, Oerskovia, Promicromonospora,* or *Intrasporangium*.

25. The method of claim 24, wherein said enzyme is derived from a microorganism which is within the genus *Nocardioides*.

26. The method of claim 25, wherein said enzyme is derived from a microorganism selected from the group consisting of *Nocardioides albus*, *Nocardioides flavus, Nocardioides fulvus, Nocardioides luteus, Nocardioides simplex*, and *Nocardioides thermolilacinus*.

27. The method of claim 26, wherein said enzyme is derived from a microorganism selected from the group consisting of *Nocardioides albus* ATCC 55424 (SC 13910), *Nocardioides albus* ATCC 55425 (SC 13911), and *Nocardioides luteus* ATCC 55426 (SC 13912).

28. The method of claims 1 or 4, wherein the taxane product obtained is employed in the preparation of a taxane bearing an acyloxy group at C-13.

29. A method for selecting a microorganism which, when contacted with a taxane containing an acyloxy group directly bonded at C-13, is capable of hydrolyzing said acyloxy group to form a hydroxyl group directly bonded at C-13, comprising the steps of:
(a) selecting a solid growth medium (i) in which the microorganism to be screened will grow, (ii) in which the starting C-13 acyloxy-bearing taxane is insoluble and thus, in admixture with the growth medium, has a cloudy appearance, and (iii) in which the desired C-13 hydroxyl-bearing taxane product, and, optionally, the desired cleaved C-13 sidechain product, are soluble and thus, in admixture with the growth medium, has a clear appearance;
(b) placing said microorganism into contact with the growth medium selected in step (a) above, into which the starting C-13 acyloxy-bearing taxane has been admixed, and under conditions allowing growth of the microorganism to occur; and
(c) observing whether a clear zone, indicating that said microorganism is capable of said hydrolysis, appears around the area in which growth of the microorganism occurs.

30. A method for selecting a microorganism which, when contacted with a taxane containing an acyloxy group directly bonded at C-13, is capable of hydrolyzing said acyloxy group to form a hydroxyl group directly bonded at C-13, comprising the steps of;
(a) selecting a solid growth medium (i) in which the microorganism to be screened will grow, (ii) in which the starting C-13 acyloxy-bearing taxane is soluble and thus, in admixture with the growth medium, has a clear appearance, and (iii) in which the desired C-13 hydroxyl-bearing taxane product, and, optionally, the desired cleaved C-13 sidechain product, are insoluble and thus, in admixture with the growth medium, has a cloudy appearance;
(b) placing said microorganism into contact with the growth medium selected in step (a) above, into which the starting C-13 acyloxy-bearing taxane has been admixed, and under conditions allowing growth of the microorganism to occur; and
(c) observing whether a cloudy zone, indicating that said microorganism is capable of said hydrolysis, appears around the area in which growth of the microorganism occurs.

31. The microorganism *Nocardioides albus* ATCC 55424 which is biologically pure.

32. The microorganism *Nocardioides albus* ATCC 55425 which is biologically pure.

33. The microorganism *Nocardioides luteus* ATCC 55426 which is biologically pure.

34. An enzyme capable of catalyzing the hydrolysis of said acyloxy group to a hydroxyl group in the method of claim 11, said enzyme being isolated from *Nocardioides albus* ATCC 55424.

35. An enzyme capable of catalysing the hydrolysis of said acyloxy group to a hydroxyl group in the method of claim 11, said enzyme being isolated from *Nocardioides albus* ATCC 55425.

36. An enzyme capable of catalyzing the hydrolysis of said acyloxy group to a hydroxyl group in the method of claim 11, said enzyme being isolated from *Nocardioides luteus* ATCC 55426.

## Patentansprüche

1. Verfahren zur Herstellung mindestens eines Taxans, das eine direkt an C-10 gebundene Hydroxylgruppe enthält, umfassend die Schritte Zusammenbringen mindestens eines Taxans, das einen direkt an C-10 gebundenen Acyloxyrest enthält, mit einem Enzym oder Mikroorganismus, das/der die Hydrolyse dieses Acyloxyrestes zu einer Hydroxylgruppe katalysieren kann, und Durchführen dieser Hydrolyse.

2. Verfahren nach Anspruch 1, wobei mindestens ein an C-10 eine Hydroxylgruppe tragendes Taxan der nachstehenden Formel I: 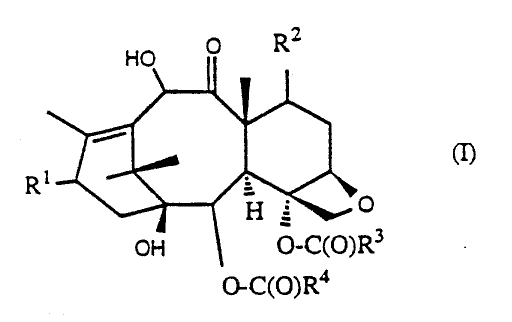 in der
R¹ eine Hydroxylgruppe oder einen Acyloxyrest bedeutet;
R² ein Wasserstoffatom, eine Hydroxylgruppe, ein Fluoratom, einen Rest R⁵-O-, eine Xylosylgruppe, einen Rest R⁶-C(O)-O- oder R⁶-O-C(O)-O- bedeutet;
R³ und R⁴ jeweils unabhängig voneinander ein Wasserstoffatom, einen Alkyl-, Alkenyl-, Alkinyl-, Cycloalkyl-, Cycloalkenyl-, Aryl- oder Heterocyclorest bedeuten;
R⁵ eine Hydroxylschutzgruppe ist; und
R⁶ ein Wasserstoffatom, einen Alkyl-, Alkenyl-, Alkinyl-, Cycloalkyl-, Cycloalkenyl-, Aryl- oder Heterocyclorest bedeutet,
oder ein Salz davon
durch Zusammenbringen mindestens eines an C-10 einen Acyloxyrest tragenden Taxans der nachstehenden Formel II: 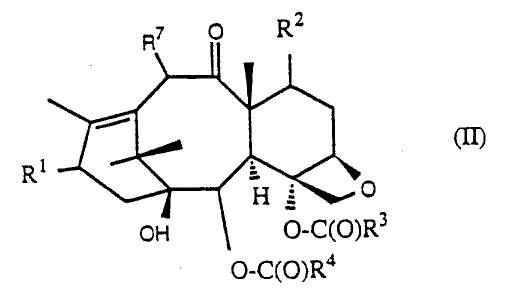 in der
R¹, R², R³ und R⁴ wie vorstehend definiert sind; und
R⁷ einen Acyloxyrest bedeutet,
oder eines Salzes davon
mit einem Enzym oder Mikroorganismus, das/der die Hydrolyse des R⁷-Acyloxyrestes zu einer Hydroxylgruppe katalysieren kann, hergestellt wird.

3. Verfahren nach Anspruch 1, wobei das im Hydrolyseverfahren eingesetzte, einen Acyloxyrest tragende Taxan-Ausgangsmaterial ein Gemisch von einem Acyloxyrest tragenden Taxanen umfasst.

4. Verfahren zur Herstellung mindestens eines Taxans, das eine direkt an C-10 gebundene Acyloxygruppe enthält, umfassend die Schritte Zusammenbringen mindestens eines Taxans, das eine direkt an C-10 gebundene Hydroxylgruppe enthält, mit einem Acylierungsmittel und einem Enzym oder Mikroorganismus, das/der die Veresterung dieser Hydroxylgruppe zur einem Acyloxyrest katalysieren kann, und Durchführen dieser Veresterung.

5. Verfahren nach Anspruch 4, wobei mindestens ein an C-10 einen Acyloxyrest tragendes Taxan der nachstehenden Formel II: 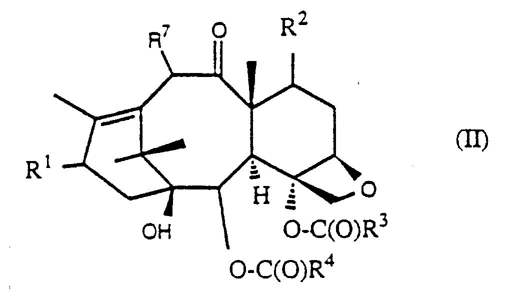 in der
R¹ eine Hydroxylgruppe oder einen Acyloxyrest bedeutet;
R² ein Wasserstoffatom, eine Hydroxylgruppe, ein Fluoratom, einen Rest R⁵-O-, eine Xylosylgruppe, einen Rest R⁶-C(O)-O- oder R⁶-O-C(O)-O- bedeutet;
R³ und R⁴ jeweils unabhängig voneinander ein Wasserstoffatom, einen Alkyl-, Alkenyl-, Alkinyl-, Cycloalkyl-, Cycloalkenyl-, Aryl- oder Heterocyclorest bedeuten;
R⁵ eine Hydroxylschutzgruppe ist;
R⁶ ein Wasserstoffatom, einen Alkyl-, Alkenyl-, Alkinyl-, Cycloalkyl-, Cycloalkenyl-, Aryl- oder Heterocyclorest bedeutet; und
R⁷ einen Acyloxyrest bedeutet,
oder ein Salz davon
durch Zusammenbringen mindestens eines an C-10 eine Hydroxylgruppe tragenden Taxans der nachstehenden Formel I: 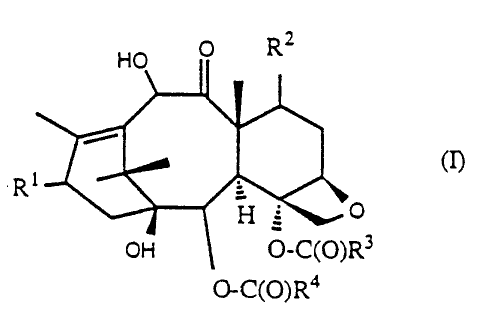 in der
R¹, R², R³ und R⁴ wie vorstehend definiert sind,
oder eines Salzes davon
mit einem Acylierungsmittel und einem Enzym oder Mikroorganismus, das/der die Veresterung der C-10-Hydroxylgruppe unter Bildung des R⁷-Acyloxyrestes katalysieren kann, hergestellt wird.

6. Verfahren nach Anspruch 4, wobei das bei der Veresterung eingesetzte, eine Hydroxylgruppe tragende Taxan-Ausgangsmaterial ein Gemisch von eine Hydroxylgruppe tragenden Taxanen umfasst.

7. Verfahren nach Anspruch 6, wobei das Gemisch von Taxanen durch Pflanzenzellkultur von und/oder Extraktion aus Pflanzengewebe erhalten wird, wobei die Pflanze ein Mitglied der Gattung *Taxus* ist.

8. Verfahren nach Anspruch 4, wobei das Acylierungsmittel eine Verbindung der nachstehenden Formel IV ist:
R¹¹-C(O)-L (IV)
in der
R¹¹ einen Alkyl-, Alkenyl-, Alkinyl-, Aryl-, Cycloalkyl-, Cycloalkenyl- oder Heterocyclorest bedeutet; und
L eine Abgangsgruppe ist, die unter Bildung einer Estergruppe verdrängt werden kann.

9. Verfahren nach Anspruch 8, wobei das Acylierungsmittel Vinylacetat ist.

10. Enzym, das die Hydrolyse des Acyloxyrestes im Verfahren nach Anspruch 1 oder die Veresterung der Hydroxylgruppe zu einem Acyloxyrest im Verfahren nach Anspruch 4 katalysieren kann, wobei das Enzym aus *Nocardioides luteus* ATCC 55426 (SC 13912) isoliert ist.

11. Verfahren zur Herstellung mindestens eines Taxans, das eine direkt an C-13 gebundene Hydroxylgruppe enthält, umfassend die Schritte Zusammenbringen mindestens eines Taxans, das einen direkt an C-13 gebundenen Acyloxyrest enthält, mit einem Enzym oder Mikroorganismus, das/der die Hydrolyse dieses Acyloxyrestes zu einer Hydroxylgruppe katalysieren kann, und Durchführen dieser Hydrolyse.

12. Verfahren nach Anspruch 11, wobei mindestens ein an C-13 eine Hydroxylgruppe tragendes Taxan der nachstehenden Formel V: 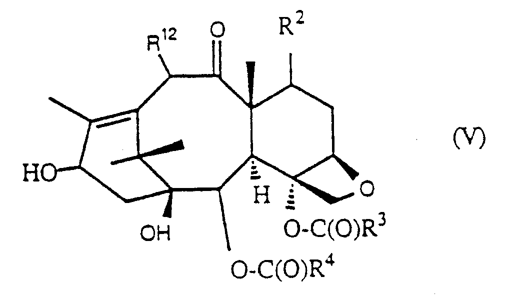 in der
R¹² ein Wasserstoffatom, eine Hydroxylgruppe, einen Rest R⁵-O-, R⁶-C(O)-O- oder R⁶-O-C(O)-O- bedeutet;
R² ein Wasserstoffatom, eine Hydroxylgruppe, ein Fluoratom, einen Rest R⁵-O-, eine Xylosylgruppe, einen Rest R⁶-C(O)-O- oder R⁶-O-C(O)-O- bedeutet;
R³ und R⁴ jeweils unabhängig voneinander ein Wasserstoffatom, einen Alkyl-, Alkenyl-, Alkinyl-, Cycloalkyl-, Cycloalkenyl-, Aryl- oder Heterocyclorest bedeuten;
R⁵ eine Hydroxylschutzgruppe ist; und
R⁶ ein Wasserstoffatom, einen Alkyl-, Alkenyl-, Alkinyl-, Cycloalkyl-, Cycloalkenyl-, Aryl- oder Heterocyclorest bedeutet,
oder ein Salz davon
durch Zusammenbringen mindestens eines an C-13 einen Acyloxyrest tragenden Taxans der nachstehenden Formel VI: 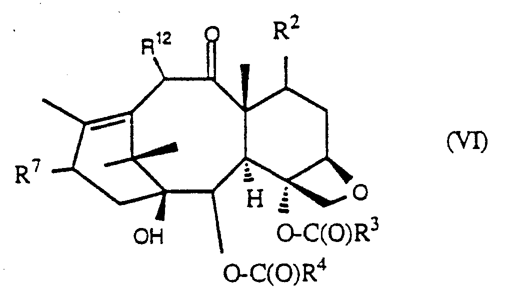 in der
R¹², R², R³ und R⁴ wie vorstehend definiert sind; und
R⁷ einen Acyloxyrest bedeutet,
oder eines Salzes davon
mit dem Enzym oder Mikroorganismus hergestellt wird.

13. Verfahren nach Anspruch 12, wobei das Taxan der Formel VI Cephalomannin, 7-Xylosyl-10-desacetyltaxol, 10-Desacetyltaxol, 7-Xylosyltaxol, Taxol-C und/oder Taxol ist und das Taxan der Formel V Baccatin III, 10-Desacetylbaccatin III, 7-Xylosyl-10-desacetylbaccatin III und/oder 7-Xylosylbaccatin III ist.

14. Verfahren nach Anspruch 12, wobei das im Hydrolyseverfahren eingesetzte, einen Acyloxyrest tragende Taxan-Ausgangsmaterial ein Gemisch von einen Acyloxyrest tragenden Taxanen mit unterschiedlichen Seitenketten an C-13 umfasst.

15. Verfahren nach Anspruch 11, wobei das Enzym eine Hydrolase ist.

16. Verfahren nach Anspruch 11, wobei nach der Hydrolyse das mindestens eine Taxan, das eine direkt an C-13 gebundene Hydroxylgruppe enthält, wobei Hydroxylgruppen an anderen Positionen als C-13 gegebenenfalls geschützt sind, mit einer Verbindung, die an C-13 eine Alkoxy-Seitenkette bildet, gekuppelt wird.

17. Verfahren nach Anspruch 16, wobei durch das Verfahren, das Hydrolyse und Kupplung umfasst, schließlich Taxol hergestellt wird.

18. Verfahren nach Anspruch 2, wobei das Taxan der Formel II Baccatin III ist und das Taxan der Formel I 10-Desacetylbaccatin III ist.

19. Verfahren nach Anspruch 3 oder 14, wobei das Gemisch von Taxanen mittels Pflanzenzellkultur von und/oder Extraktion aus Pflanzengewebe erhalten wird, wobei die Pflanze ein Mitglied der Gattung Taxus ist.

20. Verfahren nach einem der Ansprüche 1, 4 und 11, wobei ein Mikroorganismus eingesetzt wird, der einer der folgenden Gattungen angehört: *Nocardioides, Nocardia, Rhodococcus, Micropolyspora, Saccharopolyspora, Pseudonocardia, Oerskovia, Promicromonospora oder Intrasporangium.*

21. Verfahren nach Anspruch 20, wobei ein Mikroorganismus der Gattung *Nocardioides* eingesetzt wird.

22. Verfahren nach Anspruch 21, wobei der Mikroorganismus aus der Gruppe ausgewählt wird, bestehend aus *Nocardioides albus, Nocardioides flavus, Nocardioides fulvus, Nocardioides luteus, Nocardioides simplex* und *Nocardioides thermolilacinus.*

23. Verfahren nach Anspruch 22, wobei der Mikroorganismus aus der Gruppe ausgewählt ist, bestehend aus *Nocardioides albus* ATCC 55424 (SC 13910), *Nocardioides albus* ATCC 55425 (SC 13911) und *Nocardioides luteus* ATCC 55426 (SC 13912).

24. Verfahren nach einem der Ansprüche 1, 4 und 11, wobei das Enzym aus einem Mikroorganismus stammt, der einer der folgenden Gattungen angehört: *Nocardioides, Nocardia, Rhodococcus, Micropolyspora, Saccharopolyspora, Pseudonocardia, Oerskovia, Promicromonospora oder Intrasporangium*.

25. Verfahren nach Anspruch 24, wobei das Enzym von einem Mikroorganismus stammt, der der Gattung *Nocardioides* angehört.

26. Verfahren nach Anspruch 25, wobei das Enzym aus einem Mikroorganismus stammt, der aus der Gruppe ausgewählt ist, bestehend aus *Nocardioides albus, Nocardioides flavus, Nocardioides fulvus, Nocardioides luteus, Nocardioides simplex* und *Nocardioides thermolilacinus.*

27. Verfahren nach Anspruch 26, wobei das Enzym von einem Mikroorganismus stammt, der aus der Gruppe ausgewählt ist, bestehend aus *Nocardioides albus* ATCC 55424 (SC 13910), *Nocardioides albus* ATCC 55425 (SC 13911) und *Nocardioides luteus* ATCC 55426 (SC 13912).

28. Verfahren nach Anspruch 1 oder 4, wobei das erhaltene Taxan-Produkt zur Herstellung eines Taxans, das einen Acyloxyrest an C-13 trägt, eingesetzt wird.

29. Verfahren zur Selektion eines Mikroorganismus, der, wenn er mit einem Taxan mit einem direkt an C-13 gebundenen Acyloxyrest zusammengebracht wird, den Acyloxyrest hydrolysieren kann, so dass eine direkt an C-13 gebundene Hydroxylgruppe gebildet wird, umfassend die Schritte:
(a) Auswählen eines festen Wachstumsmediums, (i) in dem der zu suchende Mikroorganismus wächst, (ii) in dem das an C-13 einen Acyloxyrest tragende Ausgangs-Taxan unlöslich ist und so in der Beimischung zum Wachstumsmedium ein trübes Aussehen hat und (iii) in dem das gewünschte an C-13 eine Hydroxylgruppe tragende Taxan-Produkt und gegebenenfalls das gewünschte Produkt, bei dem die C-13-Seitenkette abgespalten ist, löslich sind und so in der Beimischung zum Wachstumsmedium ein Klares Aussehen haben;
(b) Zusammenbringen des Mikroorganismus mit dem im vorstehenden Schritt (a) ausgewählten Wachstumsmedium, in das das an C-13 einen Acyloxyrest tragende Ausgangs-Taxan gemischt worden ist, und unter Bedingungen, die das Wachstum des Mikroorganismus ermöglichen; und
(c) Beobachten, ob eine klare Zone, die anzeigt, dass der Mikroorganismus zur Hydrolyse befähigt ist, um den Bereich auftritt, in dem das Wachstum des Mikroorganismus stattfindet.

30. Verfahren zur Selektion eines Mikroorganismus, der, wenn er mit einem Taxan mit einem direkt an C-13 gebundenen Acyloxyrest zusammengebracht wird, den Acyloxyrest hydrolysieren kann, so dass eine direkt an C-13 gebundene Hydroxylgruppe gebildet wird, umfassend die Schritte:
(a) Auswählen eines festen Wachstumsmediums, (i) in dem der zu suchende Mikroorganismus wächst, (ii) in dem das an C-13 einen Acyloxyrest tragende Ausgangs-Taxan löslich ist und so in der Beimischung zum Wachstumsmedium ein klares Aussehen hat und (iii) in dem das gewünschte an C-13 eine Hydroxylgruppe tragende Taxan-Produkt und gegebenenfalls das gewünschte Produkt, bei dem die C-13-Seiterkette abgespalten ist, unlöslich sind und so in der Beimischung zum Wachstumsmedium ein trübes Aussehen haben;
(b) Zusammenbringen des Mikroorganismus mit dem im vorstehenden Schritt (a) ausgewählten Wachstumsmedium, in das das an C-13 einen Acyloxyrest tragende Ausgangs-Taxan gemischt worden ist, und unter Bedingungen, die das Wachstum des Mikroorganismus ermöglichen; und
(c) Beobachten, ob eine trübe Zone, die anzeigt, dass der Mikroorganismus zur Hydrolyse befähigt ist, um den Bereich auftritt, in dem das Wachstum des Mikroorganismus stattfindet.

31. Mikroorganismus *Nocardioides albus* ATCC 55424, der biologisch rein ist.

32. Mikroorganismus *Nocardioides albus* ATCC 55425, der biologisch rein ist.

33. Mikroorganismus *Nocardioides luteus* ATCC 55426, der biologisch rein ist.

34. Enzym, das die Hydrolyse des Acyloxyrestes zu einer Hydroxylgruppe im Verfahren nach Anspruch 11 katalysieren kann, wobei das Enzym aus *Nocardioides albus* ATCC 55424 isoliert ist.

35. Enzym, das die Hydrolyse des Acyloxyrestes zu einer Hydroxylgruppe im Verfahren nach Anspruch 11 katalysieren kann, wobei das Enzym aus *Nocardioides albus* ATCC 55425 isoliert ist.

36. Enzym, das die Hydrolyse des Acyloxyrestes zu einer Hydroxylgruppe im Verfahren nach Anspruch 11 katalysieren kann, wobei das Enzym aus *Nocardioides luteus* ATCC 55426 isoliert ist.

## Revendications

1. Méthode pour la préparation d'au moins un taxane contenant un groupe hydroxyle directement lié en C-10, comprenant les étapes de mettre en contact au moins un taxane contenant un groupe acyloxy directement lié en C-10 avec une enzyme ou un micro-organisme capable de catalyser l'hydrolyse dudit groupe acyloxy en un groupe hydroxyle, et d'effectuer ladite hydrolyse.

2. Méthode de la revendication 1, où au moins un taxane portant un hydroxyle C-10 de la formule I qui suit et préparé : 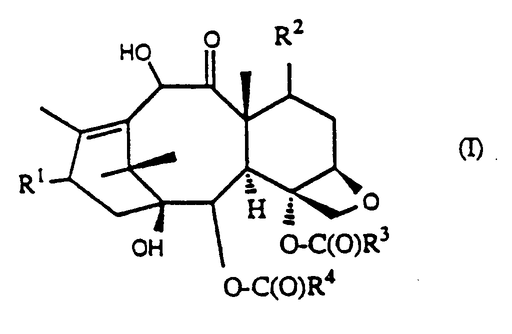 où
R¹ est hydroxyle ou acyloxy ;
R² est hydrogène, hydroxyle, fluoro, R⁵-O-, xylosyle, R⁶-C(O)-O- ou R⁶-O-C(O)-O- ;
R³ et R⁵ sont indépendamments hydrogène, alkyle, alcényle, alkynyle, cycloalkyle, cycloalcényle, aryle, ou hétérocyclo ;
R⁵ est un groupe hydroxyle protecteur ; et
R⁶ est hydrogène, alkyle, alcényle, alkynyle, cycloalkyle, cycloalcényle, aryle ou hétérocyclo,
ou un sel,
par mise en contact d'au moins un taxane portant un acyloxy C-10 de la formule II qui suit : 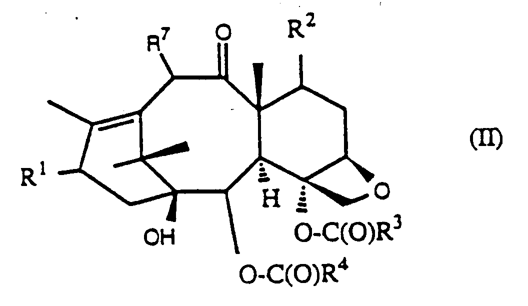 où
R¹, R², R³ et R⁴ sont tels que définis ci-dessus ; et
R⁷ est acyloxy,
ou un sel,
avec une enzyme ou un micro-organisme capable de catalyser l'hydrolyse du groupe acyloxy R⁷ en un groupe hydroxyle.

3. Méthode de la revendication 1, où la matière première de taxane portant un acyloxy que l'on emploie dans ladite méthode d'hydrolyse comprend un mélange de taxanes portant de l'acyloxy.

4. Méthode pour la préparation d'au moins un taxane contenant un groupe acyloxy directement lié en C-10, comprenant les étapes de mettre au moins un taxane contenant un groupe hydroxyle directement lié en C-10 en contact avec un agent acylant et une enzyme ou un micro-organisme capable de catalyser l'estérification dudit groupe hydroxyle en un groupe acyloxy et d'effectuer ladite estérification.

5. Méthode de la revendication 4, où au moins un taxane portant un acyloxy en C-10 de la formule II qui suit est préparé : 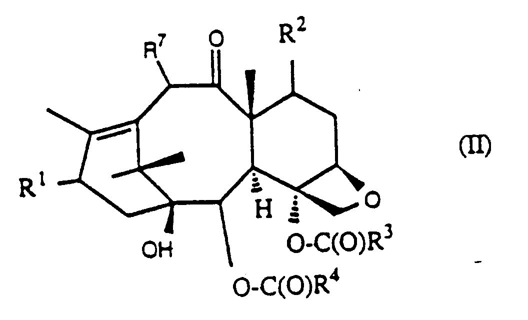 où
R¹ est hydroxyle ou acyloxy ;
R² est hydrogène, hydroxyle, fluoro, R⁵-O-, xylosyle R⁶-C(O)-O- ou R⁶-O-C(O)-O- ;
R³ et R⁴ sont indépendamments hydrogène, alkyle, alcényle, alkynyle, cycloalkyle, cycloalcényle, aryle ou hétérocyclo ;
R⁵ est un groupe hydroxyle protecteur ;
R⁶ est hydrogène, alkyle, alcényle, alkynyle, cycloalkyle, cycloalcényle, aryle ou hétérocyclo ; et
R⁷ est acyloxy,
ou un sel,
par mise en contact d'au moins un taxane portant un hydroxyle en C-10 de la formule I qui suit : 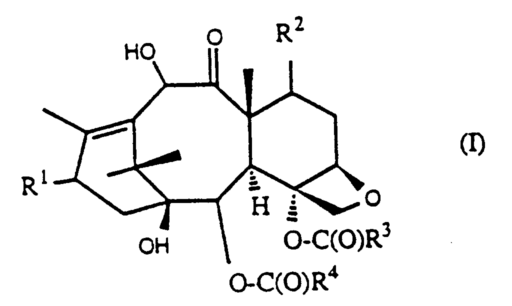 où
R¹, R², R³ et R⁴ sont tels que définis ci-dessus,
ou un sel,
avec un agent acylant et une enzyme ou un micro-organisme capable de catalyser l'estérification du groupe hydroxyle C-10 pour former ledit groupe acyloxy R⁷.

6. Méthode de la revendication 4, où la matière première de taxane portant un hydroxy employée dans ladite méthode d'estérification comprend un mélange de taxanes portant de l'hydroxy.

7. Méthode de la revendication 6, où ledit mélange de taxanes est obtenu par culture de cellules de plantes, et/ou extraction de tissues de plantes, où ladite plante est un membre du genre *Taxus*.

8. Méthode de la revendication 4, où ledit agent acylant est un composé de la formule IV qui suit :
R¹¹-C(O)-L (IV)
où
R¹¹ est alkyle, alcényle, alkynyle, aryle, cycloalkyle, cycloalcényle ou hétérocyclo; et
L est un groupe partant qui peut être déplacé pour former un groupe ester.

9. Méthode de la revendication 8, où ledit agent acylant est l'acétate de vinyle.

10. Enzyme capable de catalyser l'hydrolysis dudit groupe acyloxy dans la méthode de la revendication 1, ou capable de catalyser l'estérification dudit groupe hydroxyle en un groupe acyloxy dans la méthode de la revendication 4, ladite enzyme étant isolée de *Nocardioides luteus* ATCC 55426 (SC 13912).

11. Méthode pour la préparation d'au moins un taxane contenant un groupe hydroxyle directement lié en C-13, comprenant les étapes de mettre en contact au moins un taxane contenant un groupe acyloxy directement lié en C-13, avec une enzyme ou un micro-organisme capable de catalyser l'hydrolyse dudit groupe acyloxy en un groupe hydroxyle et d'effectuer ladite hydrolyse.

12. Méthode de la revendication 11, où au moins un taxane portant une hydroxyle C-13 de la formule V est préparé : 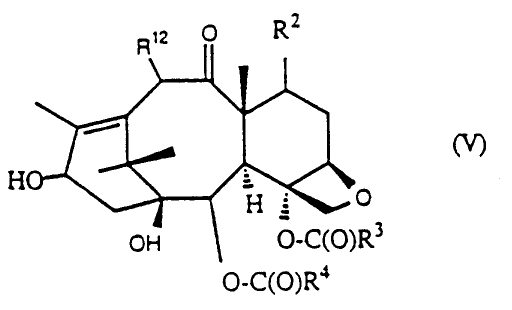 où
R¹² est hydrogène, hydroxyle, R⁵-O-, R⁶-C(O)-O- ou R⁶-O-C(O)-O- ;
R² est hydrogène, hydroxyle, fluoro, R⁵-O-, xylosyle, R⁶-C(O)-O- ou R⁶-O-C(O)-O- ;
R³ et R⁴ sont indépendamment hydrogène, alkyle, alcényle, alkynyle, cycloalkyle,cycloalcényle, aryle ou hétérocyclo ;
R⁵ est un groupe hydroxyle protecteur ; et
R⁶ est hydrogène, alkyle, alcényle, alkynyle, cycloalkyle, cycloalcényle, aryle ou hétérocyclo,
ou un sel
par mise en contact d'au moins un taxane portant un acyloxy C-13 de la formule VI qui suit : 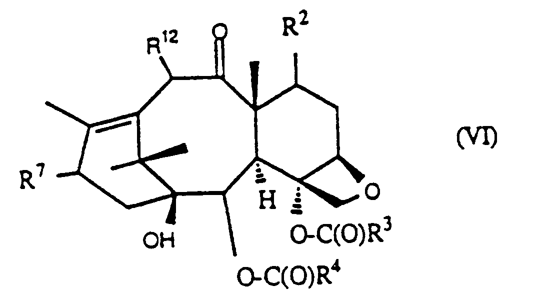 où
R¹², R², R³ et R⁴ sont tels que définis ci-dessus ; et
R⁷ est acyloxy,
ou un sel,
avec ladite enzyme ou ledit micro-organisme.

13. Méthode de la revendication 12, où ledit taxane de la formule VI est céphalomannine, 7-xylosyl-10-désacétyltaxol, 10-désacétyltaxol, 7-xylosyltaxol, taxol-C, et/ou taxol, et ledit taxane de la formule V est baccatine III, 10-désacétylbaccatine III, 7-xylosyl-10-désacétylbaccatine III et/ou 7-xylosylbaccatine III.

14. Méthode de la revendication 12, où la matière première de taxane portant un acyloxy employée dans ladite méthode d'hydrolyse comprend un mélange de taxanes portant un acyloxy ayant différentes chaînes latérales en C-13.

15. Méthode de la revendication 11, où ladite enzyme est une hydrolase.

16. Méthode de la revendication 11, où subséquemment à ladite hydrolyse, ledit au moins un taxane contenant un groupe hydroxyle directement lié en C-13, où les groupes hydroxyles aux positions autre que C-13 sont facultativement protégés, est couplé à un composé formant une chaîne latérale d'acyloxy en C-13.

17. Méthode de la revendication 16, où le taxol est finalement préparé par ladite méthode comprenant l'hydrolyse et le couplage.

18. Méthode de la revendication 2, où ledit taxane de la formule II est baccatine III et où ledit taxane de la formule I est 10-désacétylbaccatine III.

19. Méthode des revendications 3 ou 14, où ledit mélange de taxanes est obtenu par culture de cellules de plantes et/ou extraction d'un tissue de plante, où ladite plante est un membre du genre *Taxus*.

20. Méthode selon l'une quelconque des revendications 1, 4 et 11, où un micro-organisme est employé qui est dans l'un des genres suivants : *Nocardioides, Nocardia, Rhodococcus, Micropolyspora, Saccharopolyspora, Pseudonocardia, Oerskovia, Promicromonospora*, ou *Intrasporangium.*

21. Méthode de la revendication 20, où un micro-organisme est employé qui est dans le genre *Nocardioides*.

22. Méthode de la revendication 21, où ledit micro-organisme est sélectionné dans le groupe consistant en *Nocardioides albus*, *Nocardioides flavus, Nocardioides fulvus, Nocardioides luteus, Nocardioides simplex*, et *Nocardioldes thermolilacinus*.

23. Méthode de la revendication 22, où ledit micro-organisme est sélectionné dans le groupe consistant en *Nocardioides albus* ATCC 55424 (SC 13910), *Nocardioides albus* ATCC 55425 (SC 13911), et *Nocardioides luteus* ATCC 55426 (SC 13912).

24. Méthode selon l'une quelconque des revendications 1, 4 et 11, où ladite enzyme est dérivée d'un micro-organisme qui est dans l'un des genres suivants : *Nocardioides, Nocardia, Rhodococcus, Micropolyspora, Saccharopolyspora, Pseudonocardia, Oerskovia, Promicromonospora ou Intrasporangium*.

25. Méthode de la revendication 24, où ladite enzyme est dérivée d'un micro-organisme qui est dans le genre *Nocardioides*.

26. Méthode de la revendication 25, où ladite enzyme est dérivée d'un micro-organisme sélectionné dans le groupe consistant en *Nocardioides albus, Nocardioides flavus, Nocardioides fulvus, Nocardioides luteus, Nocardioides simplex, et Nocardioides thermolilacinus*.

27. Méthode de la revendication 26, où ladite enzyme est dérivée d'un micro-organisme sélectionné dans le groupe consistant en *Nocardioides albus* ATCC 55424 (SC 13910), *Nocardioides albus* ATCC 55425 (SC 13911), et *Nocardioides luteus* ATCC 55426 (SC 13912).

28. Méthode des revendications 1 ou 4, où le taxane produit obtenu est employé dans la préparation d'un taxane portant un groupe acyloxy en C- 13.

29. Méthode pour la sélection d'un micro-organisme qui, quand il est mis en contact avec un taxane contenant un groupe acyloxy directement lié en C-13, est capable d'hydrolyser ledit groupe acyloxy pour former un groupe hydroxyle directement lié en C-13, comprenant les étapes de :
(a) sélectionner un milieu de croissance solide (i) dans lequel le micro-organisme à cribler croîtra, (ii) où le taxane portant un acyloxy en C-13 de départ est insoluble et ainsi, en mélange avec le milieu de croissance, a un aspect trouble et (iii) dans lequel le taxane produit portant une hydroxyle en C-13 souhaité et facultativement, le produit de chaîne latérale en C-13 scindé souhaité, sont solubles et ainsi en mélange avec le milieu de croissance, a un aspect limpide ;
(b) placer ledit micro-organisme en contact avec le milieu de croissance sélectionné à l'étape (a) ci-desssus, où le taxane portant un acyloxy en C-13 de départ a été mélangé et dans des conditions permettant à la croissance du micro-organisme de se produire ;
(c) observer si une zone limpide, indiquant que ledit micro-organisme est capable de ladite hydrolyse, apparaît autour de la partie où se produit la croissance de micro-organisme.

30. Méthode pour la sélection d'un micro-organisme qui, quand il est mis en contact avec un taxane contenant un groupe acyloxy directement lié en C-13, est capable d'hydrolyser ledit groupe acyloxy pour former un groupe hydroxyle directement lié en C-13, comprenant les étapes de :
(a) sélectionner un milieu de croissance solide (i) dans lequel le micro-organisme à cribler croîtra, (ii) dans lequel le taxane portant un acyloxy en C-13 de départ est soluble et ainsi, en mélange avec le milieu de croissance, a un aspect limpide et (iii) dans lequel le taxane produit portant un hydroxyle en C-13 souhaité et, facultativement, le produit de chaîne latérale en C-13 scindé souhaité sont insolubles et ainsi en mélange avec le milieu de croissance, a un aspect trouble.
(b) placer ledit micro-organisme en contact avec le milieu de croissance sélectionné à l'étape (a) ci-dessus, dans lequel le taxane portant un acyloxy en C-13 de départ a été mélangé et dans des conditions permettant à la croissance du micro-organisme de se produire ; et
(c) observer si une zone trouble indiquant que ledit micro-organisme est capable de ladite hydrolyse apparaît autour de la partie dans laquelle se produit la croissance du micro-organisme.

31. Micro-organisme *Nocardioides albus* ATCC 55424 qui est biologiquement pur.

32. Micro-organisme *Nocardioides albus* ATCC 55425 qui est biologiquement pur.

33. Micro-organisme *Nocardioides luteus* ATCC 55426 qui est biologiquement pur.

34. Enzyme capable de catalyser l'hydrolyse dudit groupe acyloxy en un groupe hydroxyle dans la méthode de la revendication 11, ladite enzyme étant isolée de *Nocardioides albus* ATCC 55424.

35. Enzyme capable de catalyser l'hydrolyse dudit groupe acyloxy en un groupe hydroxyle dans la méthode de la revendication 11, ladite enzyme étant isolée de *Nocardioides albus* ATCC 55425.

36. Enzyme capable de catalyser l'hydrolyse dudit groupe acyloxy en un groupe hydroxlyle dans la méthode de la revendication 11, ladite enzyme étant isolée de *Nocardioides luteus* ATCC 55426.
